(19) Europäisches Patentamt
European Patent Office
Office européen des brevets

(11) EP 2 972 325 B1

(12) **EUROPEAN PATENT SPECIFICATION**

(45) Date of publication and mention of the grant of the patent:
**23.09.2020 Bulletin 2020/39**

(21) Application number: **14717963.4**

(22) Date of filing: **17.03.2014**

(51) Int Cl.:
*G01N 33/50* (2006.01) *G01N 33/92* (2006.01)

(86) International application number:
**PCT/US2014/030483**

(87) International publication number:
**WO 2014/145678 (18.09.2014 Gazette 2014/38)**

(54) **SYSTEM AND METHOD FOR ASSESSING QUANTITIES OR SIZES OF LIPOPROTEIN PARTICLES FROM LIPOPROTEIN PARTICLE COMPOSITIONS**

SYSTEM UND VERFAHREN ZUR BEURTEILUNG DER MENGEN ODER GRÖSSEN VON LIPOPROTEINPARTIKELN AUS LIPOPROTEINPARTIKELZUSAMMENSETZUNGEN

SYSTÈME ET MÉTHODE D'ÉVALUATION DE QUANTITÉS OU DE TAILLES DE PARTICULES DE LIPOPROTÉINES CONTENUES DANS DES COMPOSITIONS À BASE DE PARTICULES DE LIPOPROTÉINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361792539 P**

(43) Date of publication of application:
**20.01.2016 Bulletin 2016/03**

(73) Proprietor: **Helena Laboratories Corporation**
**Beaumont, TX 77704 (US)**

(72) Inventors:
- **GUADAGNO, Philip**
  **Mechanicsville, VA 23116 (US)**
- **HARRIS, William, S.**
  **Sioux Falls, SD 57105 (US)**

(74) Representative: **ARC-IP**
**ARC-IP sprl**
**Rue Emile Francqui 4**
**1435 Mont-Saint-Guibert (BE)**

(56) References cited:

| | |
|---|---|
| **EP-A1- 1 197 564** | **EP-A1- 1 197 564** |
| **EP-A2- 0 378 395** | **EP-A2- 0 378 395** |
| **US-A- 4 167 467** | **US-A- 4 167 467** |
| **US-A- 4 167 467** | |

- **RAINWATER D L ET AL: "Dramatic differences in lipoprotein composition among gray short-tailed opossums (Monodelphis domestica) fed a high cholesterol/saturated fat diet", BIOCHIMICA ET BIOPHYSICA ACTA - LIPIDS AND LIPID METABOLISM, ELSEVIER SCIENCE BV. AMSTERDAM, NL, vol. 1126, no. 2, 22 June 1992 (1992-06-22), pages 159-166, XP027222987, ISSN: 0005-2760 [retrieved on 1992-06-22]**
- **BLOOM R J ET AL: "Quantitation of lipid profiles from isolated serum lipoproteins using small volumes of human serum", CLINICAL BIOCHEMISTRY, ELSEVIER INC, US, CA, vol. 14, no. 3, 1 June 1981 (1981-06-01), pages 119-125, XP023599814, ISSN: 0009-9120, DOI: 10.1016/S0009-9120(81)90239-3 [retrieved on 1981-06-01]**
- **VADLAMUDI S ET AL: "Effects of oral combined hormone replacement therapy on plasma lipids and lipoproteins", METABOLISM, CLINICAL AND EXPERIMENTAL, W.B. SAUNDERS CO., PHILADELPHIA, PA, US, vol. 47, no. 10, 1 October 1998 (1998-10-01), pages 1222-1226, XP004538746, ISSN: 0026-0495, DOI: 10.1016/S0026-0495(98)90327-4**
- **J. S. MOSHIDES: "High density lipoprotein free cholesterol and other lipids in coronary heart disease", ARTERIOSCLEROSIS, THROMBOSIS, AND VASCULAR BIOLOGY, vol. 7, no. 3, 1 May 1987 (1987-05-01), pages 262-266, XP055128310, ISSN: 1079-5642, DOI: 10.1161/01.ATV.7.3.262**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HERMIER D ET AL: "Characterization of dietary-induced hypercholesterolemia in the chicken", BIOCHIMICA ET BIOPHYSICA ACTA - LIPIDS AND LIPID METABOLISM, ELSEVIER SCIENCE BV. AMSTERDAM, NL, vol. 1124, no. 2, 4 March 1992 (1992-03-04), pages 178-184, XP023366455, ISSN: 0005-2760, DOI: 10.1016/0005-2760(92)90095-D [retrieved on 1992-03-04]
- TIM J MCDONALD ET AL: "Lipoprotein composition in HNF1A-MODY: Differentiating between HNF1A-MODY and Type 2 diabetes", CLINICA CHIMICA ACTA, ELSEVIER BV, AMSTERDAM, NL, vol. 413, no. 9, 7 February 2012 (2012-02-07), pages 927-932, XP028472912, ISSN: 0009-8981, DOI: 10.1016/J.CCA.2012.02.005 [retrieved on 2012-02-14]
- RAINWATER D L ET AL: "Dramatic differences in lipoprotein composition among gray short-tailed opossums (Monodelphis domestica) fed a high cholesterol/saturated fat diet", BIOCHIMICA ET BIOPHYSICA ACTA - LIPIDS AND LIPID METABOLISM, ELSEVIER SCIENCE BV. AMSTERDAM, NL, vol. 1126, no. 2, 22 June 1992 (1992-06-22), pages 159-166, XP027222987, ISSN: 0005-2760 [retrieved on 1992-06-22]
- BLOOM R J ET AL: "Quantitation of lipid profiles from isolated serum lipoproteins using small volumes of human serum", CLINICAL BIOCHEMISTRY, ELSEVIER INC, US, CA, vol. 14, no. 3, 1 June 1981 (1981-06-01), pages 119-125, XP023599814, ISSN: 0009-9120, DOI: 10.1016/S0009-9120(81)90239-3 [retrieved on 1981-06-01]
- VADLAMUDI S ET AL: "Effects of oral combined hormone replacement therapy on plasma lipids and lipoproteins", METABOLISM, CLINICAL AND EXPERIMENTAL, W.B. SAUNDERS CO., PHILADELPHIA, PA, US, vol. 47, no. 10, 1 October 1998 (1998-10-01), pages 1222-1226, XP004538746, ISSN: 0026-0495, DOI: 10.1016/S0026-0495(98)90327-4
- J. S. MOSHIDES: "High density lipoprotein free cholesterol and other lipids in coronary heart disease", ARTERIOSCLEROSIS, THROMBOSIS, AND VASCULAR BIOLOGY, vol. 7, no. 3, 1 May 1987 (1987-05-01), pages 262-266, XP055128310, ISSN: 1079-5642, DOI: 10.1161/01.ATV.7.3.262
- BRAVO E ET AL: "Hepatic uptake and metabolism of free cholesterol from different lipoprotein classes. An in vivo study in the rat", BIOCHIMICA ET BIOPHYSICA ACTA - LIPIDS AND LIPID METABOLISM, ELSEVIER SCIENCE BV. AMSTERDAM, NL, vol. 1003, no. 3, 28 June 1989 (1989-06-28), pages 315-320, XP027190638, ISSN: 0005-2760 [retrieved on 1989-06-28]



- RAINWATER D L ET AL: "Dramatic differences in lipoprotein composition among gray short-tailed opossums (Monodelphis domestica) fed a high cholesterol/saturated fat diet", BIOCHIMICA ET BIOPHYSICA ACTA - LIPIDS AND LIPID METABOLISM, ELSEVIER SCIENCE BV. AMSTERDAM, NL, vol. 1126, no. 2, 22 June 1992 (1992-06-22), pages 159-166, XP027222987, ISSN: 0005-2760 [retrieved on 1992-06-22]
- BLOOM R J ET AL: "Quantitation of lipid profiles from isolated serum lipoproteins using small volumes of human serum", CLINICAL BIOCHEMISTRY, ELSEVIER INC, US, CA, vol. 14, no. 3, 1 June 1981 (1981-06-01), pages 119-125, XP023599814, ISSN: 0009-9120, DOI: 10.1016/S0009-9120(81)90239-3 [retrieved on 1981-06-01]
- VADLAMUDI S ET AL: "Effects of oral combined hormone replacement therapy on plasma lipids and lipoproteins", METABOLISM, CLINICAL AND EXPERIMENTAL, W.B. SAUNDERS CO., PHILADELPHIA, PA, US, vol. 47, no. 10, 1 October 1998 (1998-10-01), pages 1222-1226, XP004538746, ISSN: 0026-0495, DOI: 10.1016/S0026-0495(98)90327-4
- J. S. MOSHIDES: "High density lipoprotein free cholesterol and other lipids in coronary heart disease", ARTERIOSCLEROSIS, THROMBOSIS, AND VASCULAR BIOLOGY, vol. 7, no. 3, 1 May 1987 (1987-05-01), pages 262-266, XP055128310, ISSN: 1079-5642, DOI: 10.1161/01.ATV.7.3.262
- HERMIER D ET AL: "Characterization of dietary-induced hypercholesterolemia in the chicken", BIOCHIMICA ET BIOPHYSICA ACTA - LIPIDS AND LIPID METABOLISM, ELSEVIER SCIENCE BV. AMSTERDAM, NL, vol. 1124, no. 2, 4 March 1992 (1992-03-04), pages 178-184, XP023366455, ISSN: 0005-2760, DOI: 10.1016/0005-2760(92)90095-D [retrieved on 1992-03-04]
- TIM J MCDONALD ET AL: "Lipoprotein composition in HNF1A-MODY: Differentiating between HNF1A-MODY and Type 2 diabetes", CLINICA CHIMICA ACTA, ELSEVIER BV, AMSTERDAM, NL, vol. 413, no. 9, 7 February 2012 (2012-02-07), pages 927-932, XP028472912, ISSN: 0009-8981, DOI: 10.1016/J.CCA.2012.02.005 [retrieved on 2012-02-14]

## Description

The invention is defined in the appended claims. Any disclosure lying outside the scope of said claims is only intended for illustrative as well as comparative purposes. This disclosure relates to methods for assessing quantities or sizes of spherical or substantially spherical lipoprotein particles present in a biological sample. The results can be used to determine whether a subject is at increased risk for cardiovascular diseases and cardiodiabetes.

NMR is able to size and count HDL, VLDL, IDL and LDL particles, but not Lp(a) particles. NMR is at best an adequate technique and technical problems impact data accuracy. NMR is touted for its reproducibility, however the data does not compare well to the accuracy of data generated by other techniques such as gel electrophoresis. For this reason NMR sizing and particle counting may not be reliable. Gel electrophoresis is good for sizing and rough approximation of lipoprotein particle number by density staining of bands, especially for Lp(a), but not for HDL. For LDL, because there is a 1:1 stoichiometric relationship between the particle and the protein, any technique that measures the amount of ApoB can be used to calculate particle numbers in a sample.

Measurements of total cholesterol in a given sample of isolated lipoprotein subtype are also not useful for determining particle size or number. This is because the standard laboratory methods for cholesterol measurement measure both the FC in the phospholipid membrane of the particle as well as the esterified cholesterols in the center of the particle. Because the esterified cholesterols in the center are mixed with triglycerides in varying proportions dependent upon a host of genetic, dietary and disease factors, total cholesterol correlates only loosely with particle sizes and is not useful for generating clinically precise and accurate data for particle numbers.

There is no existing technology which is able to measure lipoprotein particle number for all types of spherical lipoprotein particles accurately and in a single step based on objective measurement of a sample's PL or FC content.

One aspect of the disclosure relates to a method for assessing the quantities of one or more classes or subclasses of spherical or substantially spherical lipoprotein particles present in a biological sample. The method comprises isolating one or more classes or subclasses of lipoprotein particles from the non-lipoprotein components in the biological sample. The lipoprotein particles are spherical or substantially spherical. The method also comprises the step of separating at least one of free cholesterol and phospholipid from the isolated spherical or substantially spherical lipoprotein particles. A further step comprises measuring the amount of the at least one of free cholesterol and phospholipid that has been separated from the isolated spherical or substantially spherical lipoprotein particles. The method further comprises the step of determining the quantities of the one or more classes or subclasses of spherical or substantially spherical lipoprotein particles based on the measured amount of the at least one of free cholesterol and phospholipid.

Another aspect of the disclosure relates to a method for assessing the average sizes of one or more classes or subclasses of spherical or substantially spherical lipoprotein particles present in a biological sample. The method comprises the step of isolating one or more classes or subclasses of lipoprotein particles from the non-lipoprotein components in the biological sample. The lipoprotein particles are spherical or substantially spherical. The method also comprises the step of separating at least one of free cholesterol and phospholipid from the isolated spherical or substantially spherical lipoprotein particles. A further step comprises measuring the amount of the at least one of free cholesterol and phospholipid that has been separated from the isolated spherical or substantially spherical lipoprotein particles. The method further comprises the step of determining the average sizes of the one or more classes or subclasses of spherical or substantially spherical lipoprotein particles based on the measured amount of the at least one of free cholesterol and phospholipid.

Another aspect of the disclosure relates to a method of determining whether a subject is at increased risk for at least one of cardiovascular disease and cardiodiabetes. The method comprises: a) assessing the quantities of one or more classes or subclasses of spherical or substantially spherical lipoprotein particles present in a biological sample. The assessing step comprises: isolating one or more classes or subclasses of lipoprotein particles from the non-lipoprotein components in the biological sample, wherein the lipoprotein particles are spherical or substantially spherical; separating at least one of free cholesterol and phospholipid from the isolated spherical or substantially spherical lipoprotein particles; measuring the amount of the at least one of free cholesterol and phospholipid that has been separated from the isolated spherical or substantially spherical lipoprotein particles; and determining the quantities of the one or more classes or subclasses of spherical or substantially spherical lipoprotein particles based on the measured amount of the at least one of free cholesterol and phospholipid. The method also comprises: b) comparing the determined quantities of the one or more classes or subclasses of spherical or substantially spherical lipoprotein particles to a control or reference value to determine if the subject is at an increased risk for cardiovascular disease and/or cardiodiabetes.

Another aspect of the disclosure relates to a method of determining whether a subject is at increased risk for cardiovascular disease comprising: a) assessing the average sizes of one or more classes or subclasses of spherical or substantially spherical lipoprotein particles present in a biological sample. The assessing step comprises: isolating one or more classes or subclasses of lipoprotein particles from the non-lipoprotein components in the biological sample, wherein the lipoprotein particles are spherical or substantially spherical; separating at least one of free cholesterol and phospholipid from the isolated spherical or substantially spherical lipoprotein particles; measuring the amount of the at

least one of free cholesterol and phospholipid that has been separated from the isolated spherical or substantially spherical lipoprotein particles; and determining the average sizes of the one or more classes or subclasses of spherical or substantially spherical lipoprotein particles based on the measured amount of the at least one of free cholesterol and phospholipid. The method also comprises: b) comparing the average sizes of the one or more classes or subclasses of spherical or substantially spherical lipoprotein particles to a control or reference value to determine if the subject is at an increased risk for cardiovascular disease.

Another aspect of the disclosure relates to a system for assessing quantities or sizes of one or more classes or subclasses of lipoprotein particles in a biological sample. The system comprises, optionally, an isolating module configured to isolate one or more classes or subclasses of lipoprotein particles from the non-lipoprotein components in the biological sample, or to isolate the lipoprotein particles into two or more classes or subclasses. The system comprises a separating module configured to separate at least one of free cholesterol and phospholipid from the lipoprotein particles. The system also comprises a measuring module configured to yield detectable signal from an assay indicating amount of at least one of free cholesterol and phospholipid. The system further comprises a calculating module configured to determine the quantities or sizes of the lipoprotein particles based on the measured amount of at least one of free cholesterol and phospholipid, and a predetermined parameter required by the calculation. Optionally, the system comprises a storage module configured to store output information from the calculating module. Optionally, the system comprises an output module for displaying the output information from the calculating module, or generating a report from the output information for the user.

A commercial need exists to precisely and accurately measure the number of various subclasses of lipoprotein particles in blood, because the particle numbers correlate with relative health (i.e. risk of cardiovascular events, cardiodiabetes, etc.). The purpose of the disclosure is to allow calculation of Lipoprotein Particle Numbers for all spherical lipoprotein particles (including but not limited to HDL-P, LDL-P, VLDL-P, IDL-P, Lp(a)-P and their subclasses) from lipoprotein particle composition from measurement of Free Cholesterol [FC] and Phospholipid [PL] concentrations. This disclosure overcomes problems associated with other techniques used to approximate lipoprotein particle number (including NMR and electrophoretic gels) by utilizing the mathematical laws underlying geometry of a spherical particle and the rigid stoichiometric relationship of free cholesterol to phospholipid in the phospholipid monolayer membrane "wrapper" of a spherical lipoprotein particle. Briefly, as the radius of a particle increases, the surface area increases proportionally according to the geometric formula of the relationship of the radius to the surface area of the sphere. Because free cholesterol exists in the lipid mono-layer of the lipoprotein particle surface in a constant ratio to the radius of the particle, the absolute amount of free (unesterified) cholesterol [FC] in a sample containing lipoprotein particles can be used to 1) calculate the average size of the particles (given PN and [FC]) in the sample (particle diameter, 2r), and 2) calculate the number (concentration) of lipoprotein particles (given diameter and [FC]) in a given sample. Exploiting the stoichiometry of FC and PL content of spherical lipoprotein particles to radius r of the lipoprotein particles eliminates the need for measuring both particle size and particle number by various technologies alone or in combination, and can be used on any type of spherical lipoprotein particle without regards to the specific protein components. This elegantly simple technique therefore provides significant savings in time and cost that can be achieved in the setting of the diagnostic laboratory. The accuracy of the technique also allows for improved clinical decisions on the most appropriate therapies for reduction of cardiovascular risk in a given patient.

Additional aspects, advantages and features of the disclosure are set forth in this specification, and in part will become apparent to those skilled in the art on examination of the following, or may be learned by practice of the disclosure. The disclosures disclosed in this application are not limited to any particular set of or combination of aspects, advantages and features. It is contemplated that various combinations of the stated aspects, advantages and features make up the disclosures disclosed in this application.

Figure 1 depicts the structure of a lipoprotein particle. Lipoproteins, like other phospholipid structures like micelles, adopt the most efficient energetic shape- a sphere. The surface of the sphere is composed of the hydrophilic charged polar heads of the phospholipids ("PL") (orange bumps). This outer membrane also has free unesterified cholesterol in it (yellow bumps) in a known ratio to the phospholipids. Lipoprotein particles have proteins associated with them. The protein is represented by blue coils on the surface of the lipoprotein particle. Most lipoprotein particles may have more than one protein within and on them. The only lipoprotein particle with a 1: 1 ratio of particle: protein is LDL:ApoB.

Figure 2 shows a cross sectional view of a lipoprotein particle. Polar hydrophilic PL heads are shown as orange spheres on the outside of the outer membrane; hydrophobic packed PL tails are shown as squiggles on the inside of the outer membrane. Free cholesterol molecules are also depicted as yellow spheres. The hollow portion in the middle of the lipoprotein particle is packed with various amounts of cholesterol ester and triglycerides. The cholesterol ester is represented by yellow "lollipops" and the triglycerides are shown in blue.

Figure 3 is a graph showing known cardiovascular risk levels and cutoffs for various types of lipoproteins by size and particle number.

Figure 4 is a graph showing a multivariate correlation analysis scatterplot matrix derived from data in Figures 5 and 6, showing the correlations of HDL-C to HDL-P and HDL3. Red ovals represent the 95% confidence interval for both measurements. The correlation between HDL-C and total HDL-P is 0.6877. It can be clearly seen that most of the data points that fall outside of the 95% confidence interval do so when particle count or total cholesterol measurement is high. The correlation between HDL-C and HDL3 particle number is 0.8794, which is better than the correlation of HDL-C and total HDL-P, because HDL3 have a small diameter by definition (7-9 Angstroms), as opposed to the wide variability in diameters of total HDL particles (7-13 Angstroms). However, neither correlation is good enough to allow for an accurate calculation of particle numbers from total HDL-C, or vice versa, due to errors of measurements introduced by NMR technique.

Figure 5 is a graph showing a regression plot and analysis of bivariate fit of total HDL particle numbers by HDL-C. Laboratory test results stored in a database of electronic records from 11,108 individuals were pulled at random and linear correlation analysis for bivariate fit of HDL particle number by HDL-C was performed. Particle numbers were measured with NMR technique and total HDL-C was measured by standard precipitation technique. Data demonstrate that the correlation between total HDL particle number and HDL-C is poor and that the higher the number of particles measured by NMR, the looser the correlation becomes. This demonstrates that calculation of HDL-P using measured HDL-C would be highly inaccurate, and most particularly when there are high particle numbers and/or high levels of HDL-C.

Figure 6 is a graph showing a regression plot and analysis of bivariate fit of HDL particle number by HDL3. Laboratory test results stored in a database of electronic records from 11,108 individuals were pulled at random and linear correlation analysis for bivariate fit of HDL particle number by HDL3 was performed. Particle numbers were measured with NMR technique. Data demonstrate that the correlation between total HDL particle number and HDL3 particle number as measured by NMR is least accurate when one or both particle numbers measure high. Because HDL-3 is a subset of total HDL-P, a tighter correlation would be expected since they should vary similarly in magnitude and direction in a given patient; the somewhat loose correlation can be partially explained by lack of accuracy in particle counting by NMR

The first step of this disclosure is to contact a biological sample and manipulate the sample according to various separation strategies known to those skilled in the art to obtain a pure or reasonably pure amount of a given lipoprotein species or sub-species.

Previous practice was to then determine total cholesterol content of the lipoprotein species by precipitation; this is a 2 step process in which the sample is first subjected to cholesterol esterase and then cholesterol oxidase. This liberates all cholesterol from the particles for measurement.

In this disclosure, the main concern is FC or PL in the phospholipid particle membrane, since only FC and PL have a stoichiometric relationship with both particle size and number. Therefore when the PL or FC content in a sample has been determined, and the other can be calculated. The general description of the method of determining free (or non- esterified) cholesterol content of the lipoprotein species is by precipitation or electrophoresis:

Precipitation: First using DxS04 (Dextran Sulfate Delipidized, Human Serum) to precipitate all non-HDL particles. FC can be measured from the supernatant and HDL-P calculated.

Precipitation/fractional HDL-P: Further, fractionations of HDL-P can produce supemants with various sized HDL-P for FC analysis to more accurately calculate HDL-P for specific sub-classes.

Lipid particle separation can also be accomplished with the use of detergents and/or surfactants with subsequent FC analysis.

Electrophoresis can be used to simultaneously separate all families of lipid particles and electrophoretic gels can be subsequently probed for FC. Given diameters for these particles, PN can be calculated for all lipid particles, (HDL-P, VLDL-P, IDL-P and LDL-P), simultaneously.

Using the measured amount of free cholesterol, it is possible to work backwards using Shen's formulas published in his 1977 paper (See Shen et al., "Structure of Human Serum Lipoproteins Inferred from Compositional Analysis," PNAS USA 74(3):837-41 (1977)) to calculate particle numbers.

Alternative embodiment: the same thing can be done with measurement of phospholipids which are present only in the membrane, using a different calculation from Shen's paper.

Exemplary embodiment: Calculation of HDL-P from Free Cholesterol (FC) or Phospholipid (PL) concentrations in HDL (or HDL subfractions which can be separated by precipitation, ultracentifugation or electrophoretic methods)

1. From Phospholipid concentration on HDL (or $HDL_2$ and $HDL_3$ subfractions)

    a. Measure the PL concentrations for each
    b. Use the Shen's equation 1 to calculate [HDL-P] = [PL] x (62Å$^2$**/PL molecule) x (1/4$\pi$r$^2$***)†

2. From Free Cholesterol concentration on HDL (or $HDL_2$ and $HDL_3$ subfractions)
Use Shen's equation 2 to calculate [HDL-P] = [FC] x $1/N_{fc}$ where $N_{fc}$ = free cholesterol molecules/particle

$$N_{fc} = [r^3 - (r-20.2)3]e^{(-84.4/r-6.09)} \dagger$$

a. Measure FC concentration in the biological sample of lipoprotein
b. Insert the known radii*** for $HDL_2$ or $HDL_3$
c. Solve for particle number

** specific volume for a PL molecule in the shell
***insert known radii for $HDL_2$ and $HDL_3$ ($HDL_3$ is 7-9 nm diameter and $HDL_2$ is 9-13 nm diameter). Alternatively, physically measure the average size of the particles in the biological sample and insert the radius into this equation; radius = diameter/2.
† Particle number is reported as a concentration but described as a "count" or "number."

FC measurement can be performed by any technique known in the art.

Rather than estimated radii for calculations, actual size measurements of the patients' lipoprotein particle average size obtained by any method may be substituted into the equations to derive particle number.

Measurements and derivations may be carried out on any spherical lipoprotein, not just HDLs.

Derived particle numbers (concentrations) will be related to known risk levels for cardiovascular disease, and recommendations for therapeutic management of risk will be made based on the determinings.

An additional step of compositional analysis of the lipoprotein esterified cholesterol and triglycerides may be carried out and the data related to the calculated size/particle number for risk stratification

Empirically derived algorithms determined experimentally from population studies relating FC to particle number may also be used to derive particle number for any species of lipoprotein particle, including HDL-1 and subclasses HDL-2 and HDL-3.

This disclosure utilizes previously unappreciated geometric mathematical relationships of spheres and known physical properties of FC and LP to mathematically derive lipoprotein particle numbers from a single FC measurement (and in some cases actual measured radius derived from actual measured particle size). The disclosure will allow for more accurate and precise (less "noisy") measurement of particle size overall, by eliminating the need to use inaccurate technologies to generate an actual measurement. The calculations rest on FC measurement, which is a very reproducible and accurate technique, and on known particle size ranges and/or measures of particle sizes by various technologies.

The disclosure can be used for any spherical lipoprotein particle, unlike existing technologies.

The method is a simple, rapid, automated and cost effective alternative to previous technologies. The method eliminates the need for multiple measurements. Calculations can be automated by software for report generation. Technique can be employed on all spherical lipoproteins, including the difficult HDL and LP(a) subclasses which confound other techniques. Because only FC is measured, the process of precipitating and measuring the cholesterol will not take as long; also because no cholesterol esterase needs to be added to the reaction, the cost of materials to perform the assay is less. Assay can be automated on existing robotic equipment; this eliminates the need to "send out" samples for NMR analysis or buy NMR machines, which are very large and cost $1M apiece.

The terms "quantities," "levels," "amounts," "concentrations," and "numbers" when used to describe the amount of lipoprotein particles, cholesterol, phospholipid, etc. are herein interchangeable.

The term "apolipoprotein" as used herein refers to a protein that combines with lipids to form a lipoprotein particle. Examples of apolipoprotein types are described in more detail below. The unique nature of the apolipoprotein is their stoichiometric relationship to lipoprotein particles, providing an estimate of the lipoprotein particle number, which is described in more detail below.

For the purposes of the rest of this disclosure disclosure, "cardiodiabetes" is defined as any condition related to the development and initiation of the diabetic disease process or cardiovascular disease, or complications arising therefrom, including but not limited to the following: insulin resistance, metabolic syndrome, type 2 diabetes mellitus (T2DM), type 1 diabetes mellitus (T1DM), fatty liver, diabetic nephropathy, diabetic neuropathy, vasculitis, atherosclerosis, coronary artery disease (CAD), vulnerable plaque formation, myocardial infarction (MI), cardiomyopathy, endothelial dysfunction, hypertension, occlusive stroke, ischemic stroke, transient ischemic event (TIA), deep vein thrombosis (DVT), dyslipidemia, gestational diabetes (GDM), periodontal disease, obesity, morbid obesity, chronic and acute infections, pre-term labor, diabetic retinopathy, and systemic or organ-specific inflammation.

One aspect of the disclosure relates to a method for assessing the quantities of one or more classes or subclasses of spherical or substantially spherical lipoprotein particles present in a biological sample. The method comprises isolating one or more classes or subclasses of lipoprotein particles from the non-lipoprotein components in the biological

sample. The lipoprotein particles are spherical or substantially spherical. The method also comprises the step of separating at least one of free cholesterol and phospholipid from the isolated spherical or substantially spherical lipoprotein particles. A further step comprises measuring the amount of the at least one of free cholesterol and phospholipid that has been separated from the isolated spherical or substantially spherical lipoprotein particles. The method further comprises the step of determining the quantities of the one or more classes or subclasses of spherical or substantially spherical lipoprotein particles based on the measured amount of the at least one of free cholesterol and phospholipid.

Another aspect of the disclosure relates to a method for assessing the average sizes of one or more classes or subclasses of spherical or substantially spherical lipoprotein particles present in a biological sample. The method comprises the step of isolating one or more classes or subclasses of lipoprotein particles from the non-lipoprotein components in the biological sample. The lipoprotein particles are spherical or substantially spherical. The method also comprises the step of separating at least one of free cholesterol and phospholipid from the isolated spherical or substantially spherical lipoprotein particles. A further step comprises measuring the amount of the at least one of free cholesterol and phospholipid that has been separated from the isolated spherical or substantially spherical lipoprotein particles. The method further comprises the step of determining the average sizes of the one or more classes or subclasses of spherical or substantially spherical lipoprotein particles based on the measured amount of the at least one of free cholesterol and phospholipid.

Suitable biological samples include, but are not limited to, human biological matrices, urine, plasma, serum, blood component, synovial fluid, ascitic fluid, and human lipoprotein fractions. For example, the sample may be fresh blood or stored blood or blood fractions. The sample may be a blood sample expressly obtained for the assays of this disclosure or a blood sample obtained for another purpose which can be subsampled for use in accordance with the methods described herein. For instance, the biological sample may be whole blood. Whole blood may be obtained from the subject using standard clinical procedures. The biological sample may also be plasma. Plasma may be obtained from whole blood samples by centrifugation of anti-coagulated blood. The biological sample may also be serum. The sample may be pretreated as necessary by dilution in an appropriate buffer solution, concentrated if desired, or fractionated by any number of methods including but not limited to ultracentrifugation, fractionation by fast performance liquid chromatography (FPLC), or precipitation. Any of a number of standard aqueous buffer solutions, employing one of a variety of buffers, such as phosphate, Tris, or the like, at physiological to alkaline pH can be used.

*Isolation of lipoprotein particle classes and subclasses*

As background, cholesterol and monoacylglycerols are absorbed in the intestine. Intestinal epithelial cells synthesize triacylglycerols. A portion of the cholesterol is esterified to form cholesterol esters. Intestinal cells form chylomicrons from triacylglycerols, cholesterol esters, phospholipids, free cholesterol, and apolipoproteins.

Apolipoproteins are the protein component of lipoprotein particles. Apolipoproteins coat lipoprotein particles that include cholesterol esters and triacylglyceride. The coat of the lipoprotein particle is made up of unesterified cholesterol, phospholipids, and apolipoproteins. See Figures 1 and 2. The unique nature of the apolipoprotein is their stoichiometric relationship to lipoprotein particles, providing an estimate of the lipoprotein particle number. These lipoprotein particles provide a way to circulate the hydrophobic components throughout the bloodstream. Different lipoprotein particles include chylomicron-P, VLDL-P, IDL-P, LDL-P, Lp(a)-P and HDL-P. Lipoprotein particles vary in size, shape, density, apolipoprotein composition, and lipid composition. There is heterogeneity within each class with each class sharing similar physical characteristics. By varying conditions, it is possible to visualize different particles within a class. There is clinical merit in doing so because, for example, one class may be artherogenic and one class may be artheroprotective.

Lipoprotein classes are typically heterogeneous and consisting of a set of discrete subpopulations with distinct molecular properties, including density, size, lipid composition, etc. These subpopulations of lipoprotein classes can be referred to as subclasses, subspecies, or subfractions. Depending on the methodology used to separate and analyze the lipoprotein subclasses, the number of subclasses that each lipoprotein class is divided into might be slightly different. Several different categorizations of lipoprotein subclasses by various commercial methods are shown below in Scheme 1. Harold E. Bays et al., "Once-Daily Niacin Extended Release/Lovastatin Combination Tablet Has More Favorable Effects on Lipoprotein Particle Size and Subclass Distribution Than Atorvastatin and Simvastatin," Preventive Cardiology 6(4): 179-88, 187 (2003).

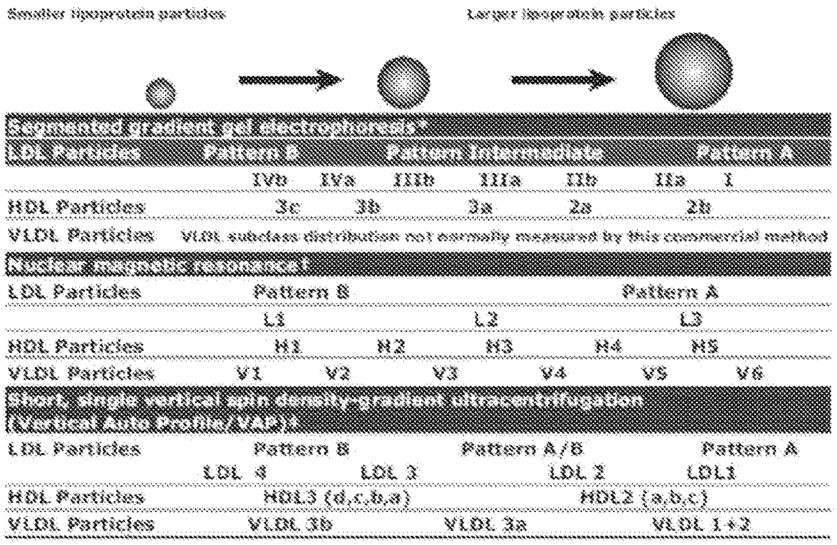


Scheme 1.

A biological sample containing one or more classes or subclasses of spherical or substantially spherical lipoprotein particles is manipulated according to various separation or isolation strategies known to one skilled in the art to isolate one or more classes or subclasses of lipoprotein particles from the non-lipoprotein contained in the sample.

The lipoprotein particles may be further isolated or separated into two or more classes, so that the subsequent separation of free cholesterol and/or phospholipid from the lipoprotein particles is performed on one or more classes of lipoprotein particles desired to be measured. For example, one class of lipoprotein particle or portions thereof can be isolated from the remaining lipoprotein classes to determine the quantities or sizes of that lipoprotein particle. Each of the remaining lipoprotein classes can be similarly and sequentially isolated to determine the quantities or sizes of each class of lipoprotein particle.

Moreover, for each class of lipoprotein particle that has been isolated, the lipoprotein particle can be further fractionated into one or more subclasses, so that the subsequent separation of free cholesterol and/or phospholipid from the lipoprotein particles is performed on one or more subclasses of the lipoprotein particle.

Lipoprotein particles or portions thereof that may be measured include, but are not limited to, Apolipoprotein A, Apolipoprotein B, Apolipoprotein C, Apolipoprotein D, Apolipoprotein E, Apolipoprotein H, lipoprotein (a) (Lp(a)), high density lipoprotein (HDL), intermediate density lipoprotein (IDL), low density lipoprotein (LDL), very low density lipoprotein (VLDL), chylomicrons, Lipoprotein X, oxidized variants or mixtures thereof. Typical lipoprotein classes or portions thereof to be measured are LP(a), Apolipoprotein B, HDL, IDL, LDL, VLDL, and chylomicron. Suitable lipoprotein subclasses to be measured include any subclass categorization shown in Scheme 1, depending on the methodology used to separate or fractionate the lipoprotein particles.

Alternatively, by using suitable isolation or separation technology, all or nearly all classes of the lipoprotein particles can be isolated from the non- lipoprotein components contained in the biological sample simultaneously, and different classes or subclasses of the lipoprotein particles can also be isolated or separated from each other simultaneously.

Lipoprotein particles that can be simultaneously isolated or separated include two or more of HDL, IDL, LDL, VLDL. Depending on the methodology used to separate or fractionate the lipoprotein particles, all subclasses of these lipoprotein classes can be simultaneously separated as well.

Isolating one or more classes or subclasses of lipoprotein particles from the non-lipoprotein, and isolating one class of the lipoprotein particles or portions thereof from the remaining lipoprotein classes can be carried out by any methods known to one skilled in the art. Suitable isolation methods include, but are not limited to, precipitation, electrophoresis, ultracentrifugation, using a surfactant, using a detergent, or combinations thereof. Similarly, fractionating a class of lipoprotein particle into one or more subclasses can also be carried out by methods known to one skilled in the art. More details about various isolation, separation, or fractionation technology for lipoprotein particles can be found in Nader Rifai et al., "Handbook of Lipoprotein Testing," (2nd Ed. American Association for Clinical Chemistry, Inc. 2001).

Precipitation can be used to isolate lipoprotein particles from the non-lipoprotein contained, and to isolate or separate different classes of lipoprotein particles from each other, with the use of appropriate reagents or solvents. Selective precipitation exploits differences in size and charge properties of the lipoproteins. Exemplary reagents are heparin-$Mn^{2+}$, dextran sulfate and $Mg^{2+}$, polyethylene glycol, and polyethylene glycol and dextran sulfate. For example, lipoprotein particles can be isolated from human serum by precipitation with polyanions, divalent cations, or heparin.

Further, a mixture of LDL and VLDL can be precipitated together and separated from the remaining lipoprotein particles. The reagents used may include heparin and divalent cations, e.g., $MnCl_2$ alone, $MnCl_2$ with sucrose, $MgCl_2$ with sodium phosphotungstate, or $MnCl_2$ with dextran sulfate. When the precipitation of LDL and VLDL is achieved with dextran sulfate and $MnCl_2$, or sodium phosphotungstate and $MgCl_2$, the HDL can subsequently be precipitated by increasing the concentrations of the reagents. Further separation of LDL and VLDL from each other and purification to remove protein contaminants can be carried out by ultracentrifugation.

Ultracentrifugation can be used to isolate lipoprotein particles from the non- lipoprotein contained, and to isolate or separate different classes of lipoprotein particles from each other, with the use of appropriate reagents or solvents, as well as to fractionate a class of lipoprotein particle into one or more subclasses. Typically, ultracentrifugation technology is based on the differences in the densities of the lipoprotein particles. Exemplary ultracentrifugation technologies are analytical ultracentrifugation, sequential density ultracentrifugation, and density gradient ultracentrifugation, using e.g., swinging bucket rotors, vertical rotors, or zonal rotors.

Chromatographic procedures, based on sizes of the lipoprotein particles, can be used to isolate lipoprotein particles from the non-lipoprotein contained, and to isolate or separate different classes of lipoprotein particles from each other, with the use of appropriate reagents or solvents, as well as to fractionate a class of lipoprotein particle into one or more subclasses. Typical chromatographic technology is liquid chromatographic, such as HPLC.

Electrophoresis technology, based on sizes of the lipoprotein particles, can be used to isolate lipoprotein particles from the non-lipoprotein contained, and to isolate or separate different classes of lipoprotein particles from each other, as well as to fractionate a class of lipoprotein particle into one or more subclasses. Typical electrophoresis technology is gel electrophoresis, such as gradient gel electrophoresis. Gel electrophoresis may be one-dimensional or two-dimensional. Isoelectric focusing may also be performed. Suitable electrophoretic gel substrates are known to those of skill in the art. For instance, suitable gel substrates include, but are not limited to, agarose or polyacrylamide. SDS-PAGE (polyacrylamide) gels separate proteins based on their size because the SDS coats the proteins with a negative charge. Separation of proteins on the agarose gel is by charge.

By electrophoresis, all or nearly all classes of the lipoprotein particles can be isolated from the non-lipoprotein components in the biological sample simultaneously, and all different classes or subclasses of the lipoprotein particles can be isolated or separated from each other simultaneously. Accordingly, the resulting electrophoretic gel for each isolated lipoprotein particle can be used for measuring the amount of the at least one of free cholesterol and phospholipid in the lipoprotein particle. In this case, the quantities or sizes of all isolated classes or subclasses of the lipoprotein particles can be determined simultaneously or nearly simultaneously. The lipoprotein particles simultaneously determined may comprise two or more different classes or subclasses of spherical or nearly spherical lipoprotein particles. For example, the lipoprotein particles simultaneously determined comprise two or more of HDL-P, VLDL-P, IDL-P, and LDL-P. All subclasses of these lipoprotein classes can be simultaneously separated as well.

Use of a detergent or surfactant to separate lipoprotein particles may be carried out as exemplified in U.S. Patent No. 6,479,249, U.S. Patent Application Publication No. 2013/0078659, and U.S. Patent Application Publication No. 2010/0227309. Surfactants and/or detergents are used regularly for the isolation of lipoprotein particles in the course of measuring particle cholesterol and triglycerides. In these methods, lipoprotein particles are selectively broken down, leaving only intact particles of interest for further analysis. Application of the same surfactant and/or detergent protocols to lipoprotein particle isolation will only change in that free cholesterol and/or phospholipids are measured instead of total cholesterol or total triglycerides. Additional steps as described herein may be used to particularly measure free cholesterol and/or phospholipids. For example, Triton™ or Tween™ detergents, or the blends of the two are useful in the isolation of lipoprotein particles for the analysis of triglycerides. The same protocols for particle isolation using Triton™ and Tween™ detergents are useful for free cholesterol and/or phospholipids measurements, with the added advantage of being easily automated. An automated protocol using detergents and/or surfactants facilitates scaling up to a high-throughput method.

Isolations of lipoprotein particle classes and subclasses for measurement of particle quantities and sizes are exemplified by isolations of HDL classes and subclasses, and LDL classes and subclasses, as described below.

HDL particles can be isolated from non-HDL particles and other non-lipoprotein components contained in the biological sample, so that at least one of free cholesterol and phospholipid separated from the isolated HDL particles can be measured to determine the quantity or size of the HDL particle. This isolation step can be carried out by precipitation with a precipitant, such as dextran sulfate. The isolated HDL particles can be further fractionated into one or more subclasses, so that at least one of free cholesterol and phospholipid separated from each fractionated HDL subclass particle can be measured to determine the quantity or size of the fractionated HDL subclass particle. For example, HDL particles can be fractionated into one or more subclasses comprise one or more of HDL- 1, HDL-2 and HDL-3. Other HDL subclasses shown in Scheme 1 can be determined, depending on the methodology used to fractionate HDL particles. This fractionation can be carried out by precipitation, electrophoresis, ultracentrifugation, using a surfactant, using a detergent, or combinations thereof. Depending on the fractionation technology used, fractionation of HDL can be carried out sequentially or simultaneously. E.g., fractionation by precipitation is carried out in sequential steps, precipitating one

HDL subclass particle at a time; and fractionation by electrophoresis can simultaneously separate all HDL subclasses from each other.

LDL particles can be isolated from non-LDL particles and other non-lipoprotein components contained in the biological sample, so that at least one of free cholesterol and phospholipid separated from the isolated LDL particles can be measured to determine the quantity or size of the LDL particle. This isolation step can be carried out by precipitation with a precipitant, such as dextran sulfate. The isolated LDL particles can be further fractionated into one or more subclasses, so that at least one of free cholesterol and phospholipid separated from each fractionated LDL subclass particle can be measured to determine the quantity or size of the fractionated LDL subclass particle. For example, LDL particles can be fractionated into one or more subclasses comprise one or more of large buoyant LDLs, small dense LDL (sdLDL), VLDL, IDL, Lp(a), and chylomicrons. Other LDL subclasses shown in Scheme 1 can be determined, depending on the methodology used to fractionate LDL particles. This fractionation can be carried out by precipitation, electrophoresis, ultracentrifugation, using a surfactant, using a detergent, or combinations thereof. Depending on the fractionation technology used, fractionation of LDL can be carried out sequentially or simultaneously. E.g., fractionation by precipitation is carried out in sequential steps, precipitating one individual LDL subclass particle at a time; and fractionation by electrophoresis can simultaneously separate all LDL subclasses from each other.

*Assessing quantities or sizes of the lipoprotein particles*

Once the lipoprotein particle (class or subclass) desired to be analyzed is isolated, the free cholesterol or phospholipid in the lipoprotein particle can be measured to determine the quantity or size of the lipoprotein particle.

Separating free cholesterol and phospholipid from the isolated spherical or substantially spherical lipoprotein particles can be carried out by any methods known to one skilled in the art. Measurement of the free cholesterol and phospholipid can also be carried out by any methods known to one skilled in the art.

Conventional practice involves determining total cholesterol content of the lipoprotein species by precipitation, which is a 2-step process that liberates all cholesterols (including free cholesterol and cholesterol ester) from the lipoprotein particles as free cholesterol for measurement. In the first step, the sample is subjected to cholesterol esterase or lipase in sufficient quantity to break down all the cholesterol esters into free cholesterol and fatty acids. In the second step, the sample is further treated with a reagent containing cholesterol oxidase (CO) to be measured spectroscopically.

Here, free cholesterol to be measured from the isolated lipoprotein particles is separated from the lipoprotein particles by treating the sample with a reagent containing cholesterol oxidase (CO), to measure the free cholesterol in the lipoprotein particles. For example, a sample of the supernatant is treated with a reagent containing CO, peroxidase (POD), 4-aminoantipyrine, and a chromogen. A quinoneimine is produced chemically in proportion to the amount of free cholesterol originally present in the supernatant; the quantity of quinoneimine produced can be measured by spectroscopically. The reaction can be shown as follows:

$$\text{Free Cholesterol} + O_2 + H_2O \xrightarrow{CO} \text{Cholest-4-en-3-one} + H_2O_2$$

$$H_2O_2 + \text{4-aminoantipyrine} + \text{chromogen} \xrightarrow{POD} \text{Quinoneimine} + H_2O$$

For example, low-sensitivity chromogens such as phenol or p-hydroxybenzoate are used; and the absorbance at about 510 nm can be measured and compared to the absorbance of reference standards at that wavelength to determine the quinoneimine concentration. This can be measured by, e.g., uv-visible absorbance spectroscopy.

In measuring the free cholesterol, unlike the conventional practice, the cholesterol esters of the lipoprotein particles are not converted into free cholesterol because only original free cholesterol in the lipoprotein particle has a stoichiometric relationship with particle sizes and numbers. Accordingly, when measuring the free cholesterol content, a cholesterol esterase is not used in separating the free cholesterol from the isolated lipoprotein particles. The esterified cholesterol in the core of the lipoprotein particle is thus not released as free cholesterol, and the measured free cholesterol only contains original free cholesterol in the lipoprotein particle, not the esterified cholesterol in the core of the lipoprotein particle.

Accordingly, the method can accurately determine the quantities and sizes of a plurality of different classes or subclasses of spherical or substantially spherical lipoprotein particles in the biological sample.

The quantities and sizes of the lipoprotein particles can be determined based on empirically derived algorithms that are determined experimentally from population studies which relate amounts of free cholesterol or phospholipid to lipoprotein particle numbers or lipoprotein particle size.

When determining the quantities of the lipoprotein particles, the radii of the spherical or substantially spherical lipoprotein particles can be predetermined. Thus, the quantities of the lipoprotein particles can be determined based on the measured amount of one of free cholesterol and phospholipid, and the predetermined radii.

For instance, a measured amount of phospholipid and the predetermined radii can be used in determining the quantities of the lipoprotein particles. In this instance, the following equation can be used:

$$\text{Particle number per unit volume} = \text{measured amount of phospholipid per unit volume in a sample} \times (62\text{Å}^2 / \text{phospholipid molecule}) \times (1/4\pi r^2).$$

Alternatively, a measured amount of free cholesterol and the predetermined radii can be used in determining the quantities of the lipoprotein particles. In this instance, the following equation can be used:

$$\text{Particle number per unit volume} = \text{quantity of measured free cholesterol per unit volume} \times 1/N_{fe},$$

wherein $N^{fc}=[r^3-(r-20.2)^3]e^{(-84.4/r-6.09)}]$

In both equations above, r is the radius of the lipoprotein particle or average radius of the class or subclass of the lipoprotein particles in Å.

The radii of the lipoprotein particles may be predetermined based on theoretical estimation. Alternatively, the radii of the lipoprotein particles may be predetermined based on experimental measurement of the average sizes of the classes and subclasses of the lipoprotein particles in an individual or in a population of individuals.

When determining the sizes of the lipoprotein particles, the particle numbers of the spherical or substantially spherical lipoprotein particles can be predetermined. Thus, the sizes of the lipoprotein particles can be determined based on the measured amount of one of free cholesterol and phospholipid, and the predetermined particle numbers.

For instance, a measured amount of phospholipid and predetermined particle numbers can be used in determining the sizes of the lipoprotein particles. In this instance, the following equation can be used:

$$\text{Particle number per unit volume} = \text{measured amount of phospholipid per unit volume in a sample} \times (62\text{Å}^2/ \text{phospholipid molecule}) \times (1/4\pi r^2).$$

r is the radius of the lipoprotein particle or average radius of the class or subclass of the lipoprotein particles in Å, and can be calculated by this equation.

Alternatively, a measured amount of free cholesterol and the predetermined particle numbers can be used in determining the sizes of the lipoprotein particles. In this instance, the following equation can be used:

$$\text{Particle number per unit volume} = \text{quantity of measured free cholesterol per unit volume} \times 1/N_{fc},$$

$$\text{wherein } N_{fc}= [r^3-(r-20.2)^3]e^{(-84.4/r-6.09)}.$$

r is the radius of the lipoprotein particle or average radius of the class or subclass of the lipoprotein particles in Å, and can be calculated by this equation.

The particle numbers of the lipoprotein particles may be predetermined based on theoretical estimation. Alternatively, the particle numbers of the lipoprotein particles may be predetermined based on experimental measurement of the particle numbers of the classes and subclasses of the lipoprotein particles in an individual or in a population of individuals.

An additional step of compositional analysis of the lipoprotein particles can be performed to determine the level of esterified cholesterol and triglycerides in the lipoprotein particles. The resulting values may be related to the calculated size/particle number of the lipoprotein particles for risk stratification.

*Determining the risk of cardiovascular diseases and cardiodiabetes*

Another aspect of the disclosure relates to a method of determining whether a subject is at increased risk for at least one of cardiovascular disease and cardiodiabetes. The method comprises: a) assessing the quantities of one

or more classes or subclasses of spherical or substantially spherical lipoprotein particles present in a biological sample. The assessing step comprises: isolating one or more classes or subclasses of lipoprotein particles from the non-lipoprotein components in the biological sample, wherein the lipoprotein particles are spherical or substantially spherical; separating at least one of free cholesterol and phospholipid from the isolated spherical or substantially spherical lipoprotein particles; measuring the amount of the at least one of free cholesterol and phospholipid that has been separated from the isolated spherical or substantially spherical lipoprotein particles; and determining the quantities of the one or more classes or subclasses of spherical or substantially spherical lipoprotein particles based on the measured amount of the at least one of free cholesterol and phospholipid. The method also comprises: b) comparing the determined quantities of the one or more classes or subclasses of spherical or substantially spherical lipoprotein particles to a control or reference value to determine if the subject is at an increased risk for cardiovascular disease and/or cardiodiabetes.

Another aspect of the disclosure relates to a method of determining whether a subject is at increased risk for cardiovascular disease comprising: a) assessing the average sizes of one or more classes or subclasses of spherical or substantially spherical lipoprotein particles present in a biological sample. The assessing step comprises: isolating one or more classes or subclasses of lipoprotein particles from the non-lipoprotein components in the biological sample, wherein the lipoprotein particles are spherical or substantially spherical; separating at least one of free cholesterol and phospholipid from the isolated spherical or substantially spherical lipoprotein particles; measuring the amount of the at least one of free cholesterol and phospholipid that has been separated from the isolated spherical or substantially spherical lipoprotein particles; and determining the average sizes of the one or more classes or subclasses of spherical or substantially spherical lipoprotein particles based on the measured amount of the at least one of free cholesterol and phospholipid. The method also comprises: b) comparing the average sizes of the one or more classes or subclasses of spherical or substantially spherical lipoprotein particles to a control or reference value to determine if the subject is at an increased risk for cardiovascular disease.

The apolipoprotein A (Apo A) family constitute the major proteins found in HDL-P and triglyceride-rich lipoprotein particles. Apo A, as part of HDL, is involved in the removal of free cholesterol from extrahepatic tissues and also plays a role in the activation of lecithin acyltransferase. Apolipoprotein A activates the enzymes driving cholesterol transfer from the tissues into HDL and is also involved in HDL recognition and receptors binding in the liver.

There are multiple forms of apolipoprotein A. The most common forms are Apo A-I and Apo A-II. Apolipoprotein A (A-I, A-II, and A-IV) are found in chylomicrons and HDL. Apo A-I is the major apolipoprotein A attached to HDL. Apo A-I is responsible for activating lecithin-cholesterol acyltransferase and Apo A-II modulates that activation. Lecithin-cholesterol acyltransferase converts free cholesterol into a cholesterol ester. Apo A- IV secretions increase when fat is absorbed in the intestines. Apo A-IV may also function in activation of lecithin-cholesterol acyltransferase.

Apo A-I is found in greater proportion than Apo A-II (about 3 to 1). Lower levels of Apo A commonly correlate with the presence of cardiovascular disease (CVD) and peripheral vascular disease. Apo A-I may be a better predictor of atherogenic risk than HDL- cholesterol (HDL-C). Certain genetic disorders cause Apo A-I deficiencies and associated low levels of HDL particles. These patients also tend to have hyperlipidemia with elevated LDL particles. This contributes to accelerated rates of atherosclerosis. Apo A levels may be extremely low in alpha lipoproteinemia (also known as familial high density lipoprotein deficiency).

The role of HDL and its major apolipoprotein Apo A-I in cholesterol efflux from macrophages has been studied extensively. While HDL competes for Apo A-I binding, Apo A-I is not a competitor for HDL binding. This observation suggests that HDL and Apo A-I are binding to macrophages at least in part by distinct receptors. For example, pre- -HDL and lipid-free Apo A-I are poor ligands for the scavenger receptor (SR-BI), explaining the lack of competition of HDL binding by Apo A-I. Conversely, it has been shown that Apo A- I can dissociate from HDL, so that lipid-free Apo A-I could be available for the competition of the Apo A-I binding site by HDL. Lorenzi et al., "Apolipoprotein A-I but not high-density lipoproteins are internalised by RAW macrophages: roles of ATP-binding cassette transporter AI and scavenger receptor." BIJMol Med. 86: 171-183 (2008). Apo A-II, another component of HDL, has been shown to be pro- atherogenic in animal models. Meyers et al., "Pharmacologic elevation of high-density lipoproteins: recent insights on mechanism of action and atherosclerosis protection." Curr Opin Cardiol. 19(4):366-373 (2004).

Apolipoprotein B (Apo B-100 and Apo B-48) is the protein component of LDL. One molecule of Apo B is present in the phospholipid layer of each LDL. Over 90% of the LDL particle is composed of Apo B. Apo B functions to solubilize cholesterol within the LDL complex, which in turn increases the transport capacity of LDL for subsequent deposit of LDL cholesterol on the arterial wall. The deposit contributes to cardiovascular disease. Apo B is also a protein component of chylomicrons, VLDL, IDL, and Lp(a). Apo B is a large amphipathic helical glycoprotein with 2 isoforms: Apo B-100 (synthesized in the hepatocytes) and Apo B-48 (the structural protein of chylomicrons). Chylomicrons contain Apo B-48 while other lipoprotein particles that contain Apo B contain Apo B-100.

Apo B modulates the activity of enzymes that act on lipoprotein particles, maintains the structural integrity of the lipoprotein particle complex, and facilitates the uptake of lipoprotein particles by acting as ligands for specific cell-surface receptors.

Enzymes that act on lipoprotein particles include, but are not limited to, lipoprotein lipase, lecithin-cholesterol acyltransferease, hepatic -triglyceride lipase, and cholesterol ester transfer protein. Elevated levels of Apo B are found in hyperlipoproteinemia. Apo B-100 is absent in forms of abetalipoproteinemia. High levels of Apo B-100 may be present in hyperlipoproteinemia, acute angina, and myocardial infarction. Apo B-48 stays in the intestine in chylomicron retention disease.

It is well established that increased plasma concentration of Apo B-containing lipoprotein particles is associated with an increased risk of developing atherosclerotic disease. Case control studies have found plasma Apo B concentrations to be more discriminating than other plasma lipids and lipoprotein particles in identifying patients with coronary heart disease (CHD). See De Backer et al., "European Guidelines on Cardiovascular Disease Prevention in Clinical Practice. Third Joint Task Force of European and other Societies on Cardiovascular Disease Prevention in Clinical Practice," Eur Heart J 24: 1601- 1610 (2003); Walldius & Jungner, "Apolipoprotein B and Apolipoprotein A-I: Risk Indicators of Coronary Heart Disease and Targets for Lipid-modifying Therapy," J Intern Med 255(2): 188-205 (2004); Walldius, et al., "The apoB/apoA-I ratio: A Strong, New Risk Factor for Cardiovascular Disease and a Target for Lipid-Lowering Therapy- A Review of the Evidence," J Intern Med. 259(5):493-519 (2006); Yusuf et al., "Effect of Potentially Modifiable Risk Factors Associated with Myocardial Infarction in 52 Countries (the TNTERHEART Study): Case-control Study," Lancet 364: 937-52 (2004). The utility of Apo B in determining CHD risk has been confirmed by prospective studies, although the extent to which Apo B concentrations were better than serum lipids in predicting risk was variable. Apo B is a component of all atherogenic or potentially atherogenic particles, including very low density lipoprotein particles (VLDL-P), intermediate density lipoprotein particles (IDL-P), low density lipoprotein particles (LDL-P), and lipoprotein(a) particles(Lp(a)-P), and each particle contains one molecule of Apo B. Therefore, Apo B provides a direct measure of the number of atherogenic lipoprotein particles in the circulation. Total Apo B is not homogeneous. Total Apo B will be influenced by its presence of Apo B in the various particles above. Measuring total Apo B alone without separating the particles does not indicate with which particle it is associated.

There is now a clear consensus that Apo B is more strongly predictive of cardiovascular disease (CVD) than low density lipoprotein cholesterol (LDL-C) and a recent consensus conference report from the American Diabetes Association (ADA) and the American College of Cardiology (ACC) recognizes the importance of measurement of Apo B (see Kannel et al., "Cholesterol in the Prediction of Atherosclerotic Disease," Ann Intern Med 90:85-91 (1979) and Jeyarajah et al., "Lipoprotein Particle Analysis by Nuclear Magnetic Resonance Spectroscopy," Clin Lab Med 26: 847-70 (2006). An elevated level of Apo B and LDL-P signifies that an individual has increased risk for cardiovascular disease. An elevated level of Apo B and Lp(a)-P signifies that an individual has increased risk for cardiovascular disease.

Further, the Apo B/Apo A-I ratio has been shown to be strongly related to risk of myocardial infarction (MI), stroke and other CV manifestations as shown in the Apolipoprotein-related mortality risk (AMORIS) (See Walldius & Jungner, "Apolipoprotein B and Apolipoprotein A-I: Risk Indicators of Coronary Heart Disease and Targets for Lipid-modifying Therapy," J Intern Med 255(2): 188-205 (2004); Walldius, et al., "The apoB/apoA-I ratio: A Strong, New Risk Factor for Cardiovascular Disease and a Target for Lipid-Lowering Therapy-A Review of the Evidence," J Intern Med. 259(5):493-519 (2006); Walldius et al., "Stroke Mortality and the Apo B/Apo A-I Ratio: Results of the AMORIS Prospective Study." J Intern Med. 259: 259-66 (2006)) and INTERHEART (Yusuf et al., "Effect of Potentially Modifiable Risk Factors Associated with Myocardial Infarction in 52 Countries (the INTERHEART Study): Case-control Study," Lancet 364: 937-52 (2004) and Yusuf et al., "Obesity and the risk of myocardial infarction in 27,000 participants from 52 countries: a case-control study, " Lancet 366: 1640-9 (2005)) studies.

Apolipoprotein C (Apo C-I, C-II, C-III) is a component of chylomicron particles, VLDL particles, IDL particles, and HDL particles. Apo C-II is an activator of lipoprotein lipase and a deficiency results in an accumulation of chylomicrons and triacylglycerols. High levels of Apo C-II are indicators of angina and myocardial infarction. Apolipoprotein C-II (Apo C-II) is a specific type of protein found in large particles absorbed from the gastrointestinal tract. It is also found in very low density lipoprotein particles (VLDL-P) which is made up of mostly cholesterol. Apo C-II is an apolipoprotein responsible for the activation of lipoprotein lipase (LPL) in capillaries and thus begins the catabolism of the chylomicron particles and VLDL-P. It is also found in HDL-P. Deficits of this Apo C-II present with grave hypertriglyceridemia and hyperchylomicronemia during fasting.

Apo C-II measurements can help to determine the specific type or cause of high blood lipids (hyperlipidemia). Persons with familial lipoprotein lipase deficiency may have high amounts of Apo C-II. Other disorders that may be associated with high Apo C-II levels include angina pectoris and heart attack. Low Apo C-II levels are seen in persons with a rare condition called familial Apo C-II deficiency.

Apolipoprotein C-III (Apo C-III) is found in very low density lipoprotein particles (VLDL-P). Apo C-III inhibits lipoprotein lipase and hepatic lipase and it is thought to inhibit hepatic uptake of triglyceride-rich particles. Apo C-IV is found in at least VLDL-P and HDL-P.

The Apo A-I, Apo C-III and Apo A-IV genes are closely linked in both rat and human genomes. The A-I and A-IV genes are transcribed from the same strand, while the A- I and C-III genes are convergently transcribed. An increase in Apo C-III levels induces the development of hypertriglyceridemia.

Apolipoprotein D is a minor component of HDL. High concentrations of Apo D are correlated with various

diseases such as gross cystic disease of the breast and Alzheimer's disease.

Apolipoprotein E (Apo E-2, E-3, and E-4) are found in chylomicrons and IDL. Apo E binds to a receptor on liver cells and peripheral cells. Apo E is essential for the normal catabolism of triglyceride-rich lipoprotein particle constituents. Apo E was initially recognized for its importance in lipoprotein particle metabolism and cardiovascular disease. It plays a role in the transport of lipids to the tissues, the transport of cholesterol from the organs to the liver, in lipoprotein particle metabolism by clearing VLDL and chylomicrons, and in formation of atherosclerotic lesions. The Apo E portion of the lipoprotein particles mediates the binding of Apo E lipoprotein particles to the LDL receptor. Apo E bound to HDL-P inhibits agonist induced platelet aggregation by binding to sites on the platelets. Three different alleles of the Apo E gene exist, Apo E e2, e3, and e4. Apo E e4 is associated with an increased risk of late onset Alzheimer's disease.

Apolipoprotein H functions to bind cardiolipin. Anti-cardiolipin antibodies are found in syphilis, sclerosis, and lupus and in some diseases the antibodies require Apo H to be active and inhibit serotonin release by the platelets and prevent aggregation of platelets. Apo H also inhibits serotonin release by platelets and prevents aggregation of platelets.

Lipoprotein particle profiles are different for different individuals and for the same individual at different times. Chylomicrons are produced in the intestine and transport digested fat to the tissues. Lipoprotein lipase hydrolyzes triacylgylcerol to form fatty acids. Chylomicrons are one of the largest buoyant particles. VLDL is formed from free fatty acids upon metabolism of chylomicrons in the liver. Lipoprotein lipase hydrolyzes triacylgylcerol to form fatty acids. IDL is the unhydrolyzed triacylglycerol of VLDL. IDL becomes LDL due to hepatic lipase. HDL plays a role in the transfer of cholesterol to the liver from peripheral tissues. HDL is synthesized in the liver and intestines.

LDL particles bind to LDL receptors. Upon receptor binding, LDL is removed from the blood. Cells use cholesterol within the LDL for membranes and hormone synthesis. LDL deposits LDL cholesterol on the arterial wall which contributes to cardiovascular disease. LDL causes inflammation when it builds up inside an artery wall. Macrophages are attracted to the inflammation and turn into foam cells when they take up LDL, causing further inflammation. Smaller, denser LDL contain more cholesterol ester than the larger, buoyant LDL.

The structure of the lipoprotein(a) particles (LP(a)-P) is that of an LDL- like particle with apolipoprotein A bound to apolipoprotein B by a disulfide bond. Lipoprotein(a) particles appear to play a role in coagulation and may stimulate immune cells to deposit cholesterol on arterial walls. A high lipoprotein(a)-P level indicates a higher risk for cardiovascular disease. Specifically, a high level for a slower migrating, more cathodic, Lp(a)-P band may be an indicator of higher risk for cardiovascular disease, as it may be associated with the smaller more atherogenic Lp(a)-P isoform Therefore, Lp(a)-P is useful in diagnostic and statistical risk assessment. Lp(a)-P may serve to facilitate LDL-P plaque deposition. Levels of Lp(a)-P are increased in atherogenic events.

Lp(a)-P may have a link between thrombosis and atherosclerosis, interfering with plasminogen function in the fibrinolytic cascade. Numerous studies have documented the relationship of high plasma Lp(a)-P concentrations to a variety of cardiovascular disorders, including peripheral vascular disease, cerebrovascular disease, and premature coronary disease. One large study of older Americans, in particular, demonstrated elevated levels of Lp(a)-P independently predict an increased risk of stroke, death from vascular disease, and death from all causes in men (see Fried et al., "The Cardiovascular Health Study: Design and Rationale," Ann. Epidemiol. 3:263-76 (1991)).

Low-density lipoprotein cholesterol, (LDL-C) has been used for measurement for assessing cardiovascular risk. However, due to the variability of HDL-C, Apo B is a better measure of circulating LDL particle number (LDL-P) and therefore a more reliable indicator of risk than that traditional LDL-C because there is 1:1 stoichiometry of Apo B and LDL particles. The sum of total Apo B includes but is not limited to the Apo B complement of LDL-P (large buoyant particles and small dense particles),
+VLDL+IDL+Lp(a)+chylomicrons. Measurement of Apo B levels and associated lipoprotein particles provides additional information on the risk of atherosclerotic heart disease beyond that of the individual measurements or the traditional LDL-C assays. Measurement of fasting plasma insulin levels and LDL particle size also provide useful information.

Oxidized variants of the above-noted lipoproteins may also be detected. Oxidized variants of lipoproteins contribute to atherogenesis, with oxidation leading to increased intracellular calcium, lowered energy production, activation of cytokines, membrane damage, all resulting in apoptosis, necrosis, and ultimately cell death. Oxidation typically begins when a reactive radical abstracts a hydrogen atom from a polyunsaturated fatty acid on the LDL particle. Lipid peroxyl and alkoxyl radicals are formed, which in turn can initiate oxidation in neighboring fatty acids, resulting in propogation of lipid peroxidation. These oxidized forms of lipoproteins are absorbed by macrophages more rapidly than the native lipoproteins and this results in macrophage cholesterol accumulation, and subsequent foam cell formation and inhibition of the motility of tissue macrophages and endothelial cells. This cascade of events results in vascular dysfunction and formation and activation of atherosclerotic lesions.

The methods for assessing the quantities or sizes of one or more classes or subclasses of spherical or substantially spherical lipoprotein particles present in a biological sample and preferred embodiments have been discussed in the above embodiments, and are all suitable for determining the risk of cardiovascular diseases and cardiodiabetes.

Once the quantities or sizes of the lipoprotein particles are determined, quantities of certain components or portions of the lipoprotein particles can also be determined based on their stoichiometric relationship to lipoprotein

particles. For example, apolipoproteins are the protein component of lipoprotein particles and have stoichiometric relationship to lipoprotein particles. Thus, the quantities of apolipoprotein can be estimated based on the assessed quantities or sizes of lipoprotein particles.

The lipoprotein particles or portions thereof to be assessed for determining the risk of cardiovascular diseases and cardiodiabetes include, but are not limited to, Apolipoprotein A, Apolipoprotein B, Apolipoprotein C, Apolipoprotein D, Apolipoprotein E, Apolipoprotein H, LP(a), HDL, IDL, LDL, VLDL, chylomicrons, Lipoprotein X, oxidized variants or mixtures thereof. Exemplary lipoprotein particles or portions thereof to be assessed are Apolipoprotein B, LP(a), HDL, IDL, LDL, VLDL, oxidized variants, or mixtures thereof.

The method for determining the risk of cardiovascular diseases or cardiodiabetes can comprise assessing the levels of the different Apolipoproteins and/or lipoprotein particles present in the biological sample. In one embodiment, at least one of the lipoprotein particles or portions thereof assessed is oxidized LDL. In one embodiment, at least one of the lipoprotein particles or portions thereof assessed is Apolipoprotein B.

The lipoprotein particles or portions thereof to be assessed for determining the risk of cardiovascular diseases and cardiodiabetes can comprise at least Apolipoprotein B and LDL. For example, an elevated level of Apolipoprotein B and LDL-P indicates that an individual has increased risk for cardiovascular diseases.

The lipoprotein particles or portions thereof to be assessed for determining the risk of cardiovascular diseases and cardiodiabetes can comprise at least Apolipoprotein B and LP(a) isoform. For example, an elevated level of Apolipoprotein B and LP(a) isoform type indicates that an individual has increased risk for cardiovascular diseases.

Determining lipid-related cardiovascular diseases and/or cardiodiabetes from their correlations with the quantities and size of the one or more classes or subclasses of lipoprotein particles or portion thereof refers to a statistical correlation of the resulting lipoprotein concentration and size distribution with population mortality and risk factors, as well known in the art. Determination in the context of monitoring cardiovascular diseases and cardiodiabetes (e.g., for responsiveness to a therapeutic intervention) refers to comparison of the lipoprotein concentration and size distribution at two time points (e.g., before and after a therapeutic intervention is conducted).

The determining may include correlating the detennined levels of the different Apolipoproteins and/or lipoprotein particles to a control or reference value to determine if the subject is at an increased risk for cardiovascular disease and/or cardiodiabetes.

The determining may also include assigning the subject to a risk category selected from the group consisting of high risk, intermediate risk, and low risk (or optimal) groups for developing or having cardiovascular disease and/or cardiodiabetes. There are well established recommendations for cut-off values for biochemical markers (for example, and without limitation, cholesterol and lipoprotein levels) for determining risk. For instance, anti- ApoB binding/detection may be correlated to cut-off estimates for assigning a risk category based on Lp(a)-P and LDL-P. For instance, the cut-off values for assigning such risk categories may be as follows: Lp(a)-P: <75nmol/L optimal, 76-125nmol/L intermediate risk, >126nmol/L high risk; LDL-P: <1000nmol/L optimal, 1000-1299nmol/L intermediate risk, >1300nmol/L high risk.

The above two or more different lipoprotein particles or portions thereof may comprise at least ApoB and LDL. An elevated level of Apolipoprotein B and LDL particles detected indicates that an individual has increased risk for cardiovascular disease. Since there is a 1:1 stoichiometry between ApoB and VLDL, an elevated ApoB is arithmetically related to VLDL-P.

The method for determining the risk of cardiovascular diseases and/or cardiodiabetes may further comprise monitoring the risk for developing cardiovascular diseases and/or cardiodiabetes. Monitoring can also assess the risk for developing cardiovascular disease and/or cardiodiabetes. This method involves determining if the subject is at an elevated risk for developing cardiovascular diseases and/or cardiodiabetes, which may include assigning the subject to a risk category selected from the group consisting of high risk, intermediate risk, and low risk (i.e., optimal) groups for developing or having cardiovascular diseases and/or cardiodiabetes. This method also involves repeating the determining if the subject is at an elevated risk for developing cardiovascular diseases and/or cardiodiabetes after a period of time (e.g., before and after therapy). The method may also involve comparing the first and second risk categories obtained at different period of time; and determining, based on the comparison, if the subject's risk for developing cardiovascular diseases and/or cardiodiabetes has increased or decreased, thereby monitoring the risk for developing cardiovascular diseases and/or cardiodiabetes.

The method for determining the risk of cardiovascular diseases and/or cardiodiabetes can comprise a further step of separating the esterified cholesterol and/or triglyceride from each isolated spherical or substantially spherical lipoprotein particle. The amount of the esterified cholesterol and/or triglyceride in each isolated lipoprotein particle can then be measured using method of measuring cholesterol or triglyceride known to one skilled in the art. This measured amount of the esterified cholesterol and/or triglyceride of the lipoprotein particles can then compared to a control or reference value to determine if the subject is at an increased risk for cardiovascular disease.

*Therapy regimen*

After the subject is determined to be at an increased risk for cardiovascular diseases and/or cardiodiabetes, a therapy/treatment regimen can be selected based on the elevated risk.

The selected therapy regimen can comprise administering drugs or supplements. For instance, the drug can be an anti-inflammatory agent, an antithrombotic agent, an antiplatelet agent, a fibrinolytic agent, a lipid reducing agent, a direct thrombin inhibitor, a glycoprotein IIb/IIIa receptor inhibitor, an agent that binds to cellular adhesion molecules and inhibits the ability of white blood cells to attach to such molecules, a calcium channel blocker, a beta-adrenergic receptor blocker, an angiotensin system inhibitor, a glitazone, a GLP-1 analog, thiazolidinedionones, biguanides, neglitinides, alpha glucosidase inhibitors, an insulin, a dipeptidyl peptidase IV inhibitor, metformin, a sulfonurea, peptidyl diabetic drugs such as pramlintide and exenatide, or combinations thereof.

A therapy regimen includes, for example, drugs or supplements. The drug or supplement may be any suitable drug or supplement useful for the treatment or prevention of diabetes and related cardiovascular disease. Examples of suitable agents include an antiinflammatory agent, an antithrombotic agent, an anti-platelet agent, a fibrinolytic agent, a lipid reducing agent, a direct thrombin inhibitor, a glycoprotein IIb/IIIa receptor inhibitor, an agent that binds to cellular adhesion molecules and inhibits the ability of white blood cells to attach to such molecules, a PCSK9 inhibitor, an MTP inhibitor, mipmercin, a calcium channel blocker, a beta-adrenergic receptor blocker, an angiotensin system inhibitor, a glitazone, a GLP-1 analog, thiazolidinedionones, biguanides, neglitinides, alpha glucosidase inhibitors, an insulin, a dipeptidyl peptidase IV inhibitor, metformin, a sulfonurea, peptidyl diabetic drugs such as pramlintide and exenatide, or combinations thereof. The agent is administered in an amount effective to treat the cardiovascular disease or disorder or to lower the risk of the subject developing a future cardiovascular disease or disorder.

A therapy regimen may also include treatment for chronic infections such as UTIs, reproductive tract infections, and periodontal disease. Therapies may include appropriate antibiotics and/or other drugs, and surgical procedures and/or dentifrice for the treatment of periodontal disease.

A therapy regimen may include referral to a healthcare specialist or related specialist based on the determining of risk levels. The determining may cause referral to a cardiologist, endocrinologist, opthamologist, lipidologist, weight loss specialist, registered dietician, "health coach", personal trainer, etc... Further therapeutic intervention by specialists based on the determining may take the form of cardiac catherization, stents, imaging, coronary bypass surgeries, EKG, Doppler, hormone testing and adjustments, weight loss regimens, changes in exercise routine, diet, and other personal lifestyle habits.

Anti-inflammatory agents include but are not limited to, Aldlofenac; Aldlometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lomoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Momiflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Glucocorticoids; Zomepirac Sodium.

Anti-thrombotic and/or fibrinolytic agents include but are not limited to, Plasminogen (to plasmin via interactions of prekallikrein, kininogens, Factors XII, XHIa, plasminogen proactivator, and tissue plasminogen activator[TPA]) Streptokinase; Urokinase: Anisoylated Plasminogen-Streptokinase Activator Complex; Pro-Urokinase; (Pro-UK); rTPA (alteplase or activase; r denotes recombinant); rPro-UK; Abbokinase; Eminase; Sreptase Anagrelide Hydrochloride; Bivalirudin; Dalteparin Sodium; Danaparoid Sodium; Dazoxiben Hydrochloride; Efegatran Sulfate; Enoxaparin Sodium; Ifetroban; Ifetroban Sodium; Tinzaparin Sodium; retaplase; Trifenagrel; Warfarin; Dextrans; Heparin.

Anti-platelet agents include but are not limited to, Clopridogrel; Sulfinpyrazone; Aspirin; Dipyridamole; Clofibrate;

Pyridinol Carbamate; PGE; Glucagon; Antiserotonin drugs; Caffeine; Theophyllin Pentoxifyllin; Ticlopidine; Anagrelide.

Lipid-reducing agents include but are not limited to, gemfibrozil, cholystyramine, colestipol, nicotinic acid, probucol lovastatin, fluvastatin, simvastatin, atorvastatin, pravastatin, cerivastatin, and other HMG-CoA reductase inhibitors.

Direct thrombin inhibitors include, but are not limited to, hirudin, hirugen, hirulog, agatroban, PPACK, thrombin aptamers.

Glycoprotein IIb/IIIa receptor inhibitors are both antibodies and non-antibodies, and include, but are not limited to, ReoPro (abcixamab), lamifiban, tirofiban.

Calcium channel blockers are a chemically diverse class of compounds having important therapeutic value in the control of a variety of diseases including several cardiovascular disorders, such as hypertension, angina, and cardiac arrhythmias. Calcium channel blockers are a heterogenous group of drugs that prevent or slow the entry of calcium into cells by regulating cellular calcium channels (REMINGTON, THE SCIENCE AND PRACTICE OF PHARMACY (Twenty-First Edition, Mack Publishing Company, 2005)). Most of the currently available calcium channel blockers belong to one of three major chemical groups of drugs, the dihydropyridines, such as nifedipine, the phenyl alkyl amines, such as verapamil, and the benzothiazepines, such as diltiazem. Other calcium channel blockers include, but are not limited to, anrinone, amlodipine, bencyclane, felodipine, fendiline, flunarizine, isradipine, nicardipine, nimodipine, perhexilene, gallopamil, tiapamil and tiapamil analogues (such as 1993RO-11-2933), phenytoin, barbiturates, and the peptides dynorphin, omega-conotoxin, and omega-agatoxin, and the like and/or pharmaceutically acceptable salts thereof.

Beta-adrenergic receptor blocking agents are a class of drugs that antagonize the cardiovascular effects of catecholamines in angina pectoris, hypertension, and cardiac arrhythmias. Beta-adrenergic receptor blockers include, but are not limited to, atenolol, acebutolol, alprenolol, beftunolol, betaxolol, bunitrolol, carteolol, celiprolol, hedroxalol, indenolol, labetalol, levobunolol, mepindolol, methypranol, metindol, metoprolol, metrizoranolol, oxprenolol, pindolol, propranolol, practolol, practolol, sotalolnadolol, tiprenolol, tomalolol, timolol, bupranolol, penbutolol, trimepranol, 2-(3-(1, 1-dimethylethyl)- amino-2-hydroxypropoxy)-3-pyridenecarbonitrilHCl, 1-butylamino-3-(2,5-dichlorophenoxy-)-2-propanol, 1 -isopropylamino-3 -(4-(2-cyclopropylmethoxyethyl)phenoxy)-2-propanol, 3 - isopropylamino-1 -(7-methylindan-4-yloxy)-2-butanol, 2-(3-t-butylamino-2 -hydroxy - propylthio)-4-(5-carbamoyl-2-thienyl)thiazol, 7-(2-hydroxy-3-t-butylaminpropoxy)phthalide. The above - identified compounds can be used as isomeric mixtures, or in their respective levorotating or dextrorotating form.

An angiotensin system inhibitor is an agent that interferes with the function, synthesis or catabolism of angiotensin II. These agents will be known to those of ordinary skill in the art and include, but are not limited to, angiotensin-converting enzyme ("ACE") inhibitors, angiotensin II antagonists, angiotensin II receptor antagonists, agents that activate the catabolism of angiotensin II, and agents that prevent the synthesis of angiotensin I from which angiotensin II is ultimately derived. The renin-angiotensin system is involved in the regulation of hemodynamics and water and electrolyte balance. Factors that lower blood volume, renal perfusion pressure, or the concentration of Na+ in plasma tend to activate the system, while factors that increase these parameters tend to suppress its function.

Angiotensin (renin-angiotensin) system inhibitors are compounds that act to interfere with the production of angiotensin II from angiotensinogen or angiotensin I or interfere with the activity of angiotensin II. Such inhibitors are well known to those of ordinary skill in the art and include compounds that act to inhibit the enzymes involved in the ultimate production of angiotensin II, including renin and ACE. They also include compounds that interfere with the activity of angiotensin II, once produced. Examples of classes of such compounds include antibodies (e.g., to renin), amino acids and analogs thereof (including those conjugated to larger molecules), peptides (including peptide analogs of angiotensin and angiotensin I), pro-renin related analogs, etc. Among the most potent and useful renin-angiotensin system inhibitors are renin inhibitors, ACE inhibitors, and angiotensin II antagonists, which will be known to those of skill in the art.

Examples of drugs that act to interfere with PSK9's interaction with LDL receptors includes Aln-PCS (Alnylam); REG 727 (Regeneron); and AMG-145 (Amgen).

The drugs and/or supplements (i.e., therapeutic agents) can be administered via any standard route of administration known in the art, including, but not limited to, parenteral (e.g., intravenous, intraarterial, intramuscular, subcutaneous injection, intrathecal), oral (e.g., dietary), topical, transmucosal, or by inhalation (e.g., intrabronchial, intranasal or oral inhalation, intranasal drops). Typically, oral administration is the preferred mode of administration.

A therapy regimen may also include giving recommendations on making or maintaining lifestyle choices useful for the treatment or prevention of diabetes and cardiovascular disease based on the results of the increased risk, determined from the quantities and sizes of the lipoprotein particles or portions thereof and their associated risk levels in the subject. The lifestyle choices can involve changes in diet, changes in exercise, reducing or eliminating smoking, or a combination thereof. For example, the therapy regimen may include glucose control, lipid metabolism control, weight loss control, and smoking cessation. As will be understood, the lifestyle choice is one that will affect risk for developing or having a cardiovascular disease or disorder (see Haskell et al., "Effects of Intensive Multiple Risk Factor Reduction on Coronary Atherosclerosis and Clinical Cardiac Events in Men and Women with Coronary Artery Disease," Circulation 89(3):975-990 (1994); Ornish et al., "Intensive Lifestyle Changes for Reversal of Coronary Heart Disease," JAMA 220(23):

2001-2007 (1998); and Wister et al., "One-year Follow-up of a Therapeutic Lifestyle Intervention Targeting Cardiovascular Disease Risk," CMAJ 177(8):859-865 (2007)).

The recommendations may be provided by a health care provider such as a physician, nurse, health consultant, dietician or other trained health professional. The health care provider can repeat interaction with a patient after a period of time to reinforce recommendations and monitor progress.

Reports based on the results of determining the subject's diabetes and related cardiovascular disease risk may be generated. The reports may include suggested therapy regimens selected based on the subject's diabetes and cardiovascular disease risk. This report may be transmitted or distributed to a patient's doctor or directly to the patient. Following transmission or distribution of the report, the subject may be coached or counseled based on the therapy recommendations.

Methods according to the disclosure may also involve administering the selected therapy regimen to the subject. Accordingly, the disclosure also relates to methods of treating a subject to reduce the risk of a cardiovascular disease or disorder.

Treating the subject involves administering to the subject an agent suitable to treat a diabetes, or cardiovascular disease or disorder or to lower the risk of a subject developing a future diabetes or cardiovascular disease or disorder. Suitable agents include an anti-inflammatory agent, an antithrombotic agent, an anti-platelet agent, a fibrinolytic agent, a lipid reducing agent, a direct thrombin inhibitor, a glycoprotein IIb/IIIa receptor inhibitor, an agent that binds to cellular adhesion molecules and inhibits the ability of white blood cells to attach to such molecules, a PCSK9 inhibitor, an MTP inhibitor, mipmercin, a calcium channel blocker, a beta-adrenergic receptor blocker, an angiotensin system inhibitor, a glitazone, a GLP-1 analog, thiazolidinedionones, biguanides, neglitinides, alpha glucosidase inhibitors, an insulin, a dipeptidyl peptidase IV inhibitor, metformin, a sulfonurea, peptidyl diabetic drugs such as pramlintide and exenatide, or combinations thereof. The agent is administered in an amount effective to treat the cardiovascular disease or disorder or to lower the risk of the subject developing a future cardiovascular disease or disorder.

A therapy regimen may also include treatment for chronic infections such as UTIs, reproductive tract infections, and periodontal disease. Therapies may include appropriate antibiotics and/or other drugs, and surgical procedures and/or dentifrice for the treatment of periodontal disease.

A therapy regimen may include referral to a healthcare specialist or related specialist based on the determining of risk levels. The determining may cause referral to a cardiologist, endocrinologist, opthamologist, lipidologist, weight loss specialist, registered dietician, "health coach", personal trainer, etc. Further therapeutic intervention by specialists based on the determining may take the form of cardiac catherization, stents, imaging, coronary bypass surgeries, EKG, Doppler, hormone testing and adjustments, weight loss regimens, changes in exercise routine, diet, and other personal lifestyle habits.

Monitoring can also assess the risk for developing diabetes and cardiovascular disease. This method involves determining if the subject is at an elevated risk for developing diabetes and cardiovascular disease, which may include assigning the subject to a risk category selected from the group consisting of high risk, intermediate risk, and low risk (i.e., optimal) groups for developing or having diabetes or cardiovascular disease. This method also involves repeating the determining if the subject is at an elevated risk for developing diabetes and cardiovascular disease after a period of time (e.g., before and after therapy). The method may also involve comparing the first and second risk categories obtained at different period of time, and determining, based on the comparison, if the subject's risk for developing diabetes and cardiovascular disease has increased or decreased, thereby monitoring the risk for developing diabetes and cardiovascular disease.

*System for assessing lipoprotein particles and risk of cardiovascular diseases*

The methods described herein may be implemented using any device capable of implementing the methods. Examples of devices that may be used include but are not limited to electronic computational devices, including computers of all types. When the methods are implemented in a computer, the computer program that may be used to configure the computer to carry out the steps of the methods may be contained in any computer readable medium capable of containing the computer program.

For example, the computer system for assessing quantities or sizes of one or more classes or subclasses of lipoprotein particles in a biological sample can comprise optionally, an isolating module configured to isolate one or more classes or subclasses of lipoprotein particles from the non-lipoprotein components in the biological sample, or to isolate the lipoprotein particles into two or more classes or subclasses. The computer system can comprise a separating module configured to separate at least one of free cholesterol and phospholipid from the lipoprotein particles. The computer system can also comprise a measuring module configured to yield detectable signal from an assay indicating amount of at least one of free cholesterol and phospholipid. The computer system can further comprise a calculating module configured to determine the quantities or sizes of the lipoprotein particles based on the measured amount of at least one of free cholesterol and phospholipid, and a predetermined parameter required by the calculation. Optionally, the

computer system can comprise a storage module configured to store output information from the calculating module. Optionally, the computer system can comprise an output module for displaying the output information from the calculating module, or generating a report from the output information for the user.

The measuring module or separating module may comprise an assay that is automated on robotic equipment.

The calculating module may comprise a software to automate the determination of quantities or sizes of the lipoprotein particles.

The calculating module may also comprise a software to use empirically-derived algorithm that is determined experimentally from population studies relating quantities or sizes of at least one of free cholesterol and phospholipid to lipoprotein particle sizes or numbers, to calculate predetermined parameters (e.g., particle size, when assessing quantities of the lipoprotein particles; and particle number when assessing lipoprotein particle size).

The computer program, including the reference levels or sizes of different classes and/or subclasses of lipoprotein particles and cardiovascular factors, and predetermined parameters (e.g., predetermined particle sizes and/or particle numbers) may be contained in a computer readable medium. Examples of computer readable medium that may be used include but are not limited to diskettes, CD-ROMs, DVDs, ROM, RAM, and other memory and computer storage devices.

The computer system that may be used to configure the computer to carry out the steps of the methods may also be provided over an electronic network, for example, over the internet, world wide web, an intranet, or other network. It can also be downloaded to a computer or other electronic device such as a laptop, smart-phone, ipad, or the IT network in a provider's office. An exemplary application that carries out the steps of the methods downloadable to a computer or a smart-phone (such as iphone or ipad) has been described in details in U.S. Provisional Application No. 61/747,505, entitled, "Biomarker Bliki," filed December 31, 2012 and U.S. Patent Application No. 14/144,269, filed December 30, 2013.

References:

1. U.S. Application Publication No. 2008/0038829 A1 Process for the determination of lipoproteins in body fluids. Kremer et al./LipoFIT Analytic GhmbH.
2. U.S. Application No. 12/861.829 Assay for Determination of levels of Lipoprotein Particles in Bodily Fluids Guadagno et al./Helena HDL Inc. (including Particle size)
3. Circulation. 2002; 106: 1930-1937; LDL Particle concentration and size as determined by NMR spectroscopy as predictors of CVD in Women
4. Clin Chem 54:8; 1307-1316 (2008). Direct determination of lipoprotein particle sizes and concentrations by Ion Mobility Analysis
5. ClinChem 2004.032383 Tech Briefs. Rapid, Simple Laser-Light Scattering method for HDL particle sizing in whole plasma
6. Circulation. 2009;119:2396-2404. Advanced Lipoprotein Testing andsubfractionation are not (yet) ready for routine clinical use. (provides references for gradient gel and ultracentrifugation protocols).
7. U.S. Provisional Application Nos. 61/651,975 and 61/770,406 to Health Diagnostic Laboratory, Inc, titled "FLUORESCENT IN-SITU DETECTION OF LIPID PARTICLE APOLIPOPROTEINS WITHIN PRIMARY ELECTROPHORETIC MATRIX."
8. U.S. Provisional Application Nos. 61/779,567 and 61/652,608 to Health Diagnostic Laboratory, Inc, titled "COMPOSITION AND METHOD FOR IN SITU CALIBRATION FOR GEL ELECTROPHORESIS."

Table 1. Proof of concept data. For 3 groups of NMR particles numbers measured by NMR (low < 25, intermediate 33-34, and high > 42), 5 human serum samples per group were selected at random from blood draws on October 9-10, 2012. For each sample, FC was measured. Using the measured FC amount in total HDL-P and an average particle diameter for HDL-P, the number of HDL-P was estimated using the calculations described in this disclosure and the calculated HDL-P values were compared to the actual NMR measurements of HDL-P for the samples. Results show excellent concordance between calculated HDL-P and NMR-measurements of HDL-P, demonstrating technical feasibility of the disclosure described herein. indicates a single outlier, and interestingly this patient sample had the highest FC value of any of the 15 samples tested, indicating that the HDL-P count should theoretically be the highest in this group, therefore suggesting that the calculated particle number varies in the right direction and may be more accurate than the NMR

| Group (Particle Number) | NMR Measurement | Calculated Particle Size |
|---|---|---|
| **Low < 25** | 21 | 21.0 |

(continued)

| Group (Particle Number) | NMR Measurement | Calculated Particle Size |
|---|---|---|
| | 21 | 15.4 |
| | 21 | 22.2 |
| | 23 | 22.5 |
| | 22 | 22.0 |
| **Intermediate 33-34** | 33 | 29.2 |
| | 34 | 25.7 |
| | 34 | 31.7 |
| | 34 | 69.5* |
| | 34 | 33.9 |
| **High > 42** | 48 | 48.9 |
| | 55 | 81.4 |
| | 42 | 52.6 |
| | 47 | 45.4 |
| | 47 | 39.8 |

## Claims

1. A method for assessing the quantities of one or more of high density lipoprotein (HDL), low density lipoprotein (LDL), very low density lipoprotein (VLDL), intermediate density lipoprotein (IDL), and lipoprotein (a) (Lp(a))_lipoprotein particles present in a biological sample, comprising:

   isolating the HDL, LDL, VLDL, IDL, and Lp(a) lipoprotein particles from the non-lipoprotein components in the biological sample, to generate isolated HDL, LDL, VLDL, IDL, and Lp(a) lipoprotein particles;
   separating at least one of free cholesterol and phospholipid from the isolated HDL, LDL, VLDL, IDL, and Lp(a) lipoprotein particles;
   measuring the amount of the at least one of free cholesterol and phospholipid that has been separated from one or more of the isolated HDL, LDL, VLDL, IDL, and Lp(a) lipoprotein particles; and
   determining the quantities of one or more of HDL, LDL, VLDL, IDL, and Lp(a) lipoprotein particles based on lipoprotein particle diameter or radii and the measured amount of free cholesterol or phospholipid.

2. The method of claim 1,

   (i) wherein the method further comprises, prior to the separating step, isolating the lipoprotein particles into two or more classes and/or subclasses, wherein the subsequent separating step is performed on one or more lipoprotein particles in one or more of the isolated classes and/or subclasses,
   (ii) wherein separating free cholesterol from the isolated HDL, LDL, VLDL, IDL, and Lp(a)_lipoprotein particles is carried out by subjecting the lipoprotein particles to a cholesterol oxidase, or wherein a cholesterol esterase is not used in separating free cholesterol from the isolated HDL, LDL, VLDL, IDL, and Lp(a) lipoprotein particles, whereby esterified cholesterol in the core of the lipoprotein particle is not released as free cholesterol,
   (iii) wherein the biological sample is human biological matrix, human lipoprotein fraction, blood component, urine, plasma, serum, synovial fluid, or ascitic fluid.

3. The method of claim 2, wherein the step of isolating the lipoprotein particles into two or more classes and/or subclasses comprises:
   isolating HDL particles from non-HDL particles, wherein the at least one of free cholesterol and phospholipid separated from the isolated HDL particles is measured to determine the quantities of the HDL particles.

4. The method of claim 3, wherein the HDL particles are isolated from the non-HDL particles by precipitation with a precipitant, such as dextran sulfate.

5. The method of claim 3, further comprising
fractionating the isolated HDL particles into one or more subclasses, wherein the at least one of free cholesterol and phospholipid separated from each fractionated HDL subclass particle is measured to determine the quantity of the fractionated HDL subclass particle,
optionally wherein the HDL subclasses comprise one or more of HDL-1, HDL-2 and HDL-3.

6. The method of claim 1 or claim 5, wherein either or both of the isolating steps, or the fractionating, is carried out by precipitation, electrophoresis, centrifugation, ultracentrifugation, using a surfactant, using a detergent, or a combination thereof,
optionally wherein fractionating by precipitation is carried out in sequential steps, precipitating one HDL subclass particle at a time.

7. The method of claim 1, wherein isolating lipoprotein particles is carried out by electrophoresis to isolate all or nearly all classes of the lipoprotein particles from the non-lipoprotein components in the biological sample, and to isolate different classes or subclasses of the lipoprotein particles from each other simultaneously, and,
optionally wherein the resulting electrophoretic gel for each isolated lipoprotein particle is used for measuring amount of the at least one of free cholesterol and phospholipid in the lipoprotein particle, whereby the quantities of all isolated classes or subclasses of the lipoprotein particles can be determined simultaneously or nearly simultaneously, and,
optionally wherein the lipoprotein particles simultaneously determined comprises two or more different classes or subclasses of lipoprotein particles, or wherein the lipoprotein particles simultaneously determined comprises two or more of HDL-P, VLDL-P, IDL-P, and LDL-P.

8. The method of claim 1, wherein the determining step is based on empirically derived algorithms determined experimentally from population studies relating amounts of at least one of free cholesterol and phospholipid to lipoprotein particle numbers.

9. The method of claim 1, wherein the diameter or radii of the lipoprotein particles are predetermined; and the determining step is based on the measured amount of at least one of free cholesterol and phospholipid and the predetermined diameter or radii, optionally wherein the diameter or radii of the lipoprotein particles are predetermined based on theoretical estimation or are predetermined based on experimental measurement of the average sizes of the classes and subclasses of the lipoprotein particles in an individual or in a population of individuals.

10. The method of claim 2(i), wherein the step of isolating the lipoprotein particles into two or more classes and/or subclasses comprises:
isolating LDL particles from non-LDL particles, wherein the at least one of free cholesterol and phospholipid separated from the isolated LDL particles is measured to determine the quantities of the LDL particles; optionally further comprising:
fractionating the isolated LDL particles into one or more subclasses, wherein the at least one of free cholesterol and phospholipid separated from each fractionated LDL subclass particle is measured to determine the quantity of the fractionated LDL subclass particle.

## Patentansprüche

1. Verfahren zur Beurteilung der Mengen von einem oder mehreren Lipoproteinpartikeln aus Lipoprotein hoher Dichte (HDL), Lipoprotein niedriger Dichte (LDL), Lipoprotein sehr niedriger Dichte (VLDL), Lipoprotein mittlerer Dichte (IDL) und Lipoprotein (a) (Lp(a)), die in einer biologischen Probe vorliegen, umfassend:

   Isolieren der HDL-, LDL-, VLDL-, IDL- und Lp(a)-Lipoproteinpartikel aus den nicht-Lipoproteinbestandteilen in der biologischen Probe, um isolierte HDL-, LDL-, VLDL-, IDL- und Lp(a)-Lipoproteinpartikel zu erzeugen;
   Abtrennen mindestens eines aus freiem Cholesterin und Phospholipid von den isolierten HDL-, LDL-, VLDL-, IDL- und Lp(a)-Lipoproteinpartikeln;
   Messen der Menge von mindestens einem aus freiem Cholesterin und Phospholipid, welches von einem oder mehreren aus den isolierten HDL-, LDL-, VLDL-, IDL- und Lp(a)-Lipoproteinpartikeln abgetrennt wurde; und
   Bestimmen der Menge von einem oder mehreren aus HDL-, LDL-, VLDL-, IDL- und Lp(a)-Lipoproteinpartikeln

auf Grundlage von Lipoproteinpartikeldurchmesser oder -radien und der gemessenen Menge an freiem Cholesterin oder Phospholipid.

2. Verfahren nach Anspruch 1,

(i) wobei das Verfahren weiter vor dem Abtrennschritt das Isolieren der Lipoproteinpartikel in zwei oder mehrere Klassen und/oder Unterklassen umfasst, wobei der anschließende Abtrennschritt an einem oder mehreren Lipoproteinpartikeln in einer oder mehreren der isolierten Klassen und/oder Unterklassen ausgeführt wird,
(ii) wobei das Abtrennen des freien Cholesterins von den isolierten HDL-, LDL-, VLDL-, IDL- und Lp(a)-Lipoproteinpartikeln durch Aussetzen der Lipoproteinpartikel an eine Cholesterinoxidase ausgeführt wird oder wobei beim Abtrennen des freien Cholesterins von den isolierten HDL-, LDL-, VLDL-, IDL-und Lp(a)- Lipoproteinpartikeln keine Cholesterinesterase verwendet wird, wodurch in dem Kern der Lipoproteinpartikel kein verestertes Cholesterin als freies Cholesterin freigesetzt wird,
(iii) wobei die biologische Probe menschliche biologische Matrix, menschliche Lipoproteinfraktion, Blutbestandteile, Urin, Plasma, Serum, Synovialflüssigkeit oder Aszitesflüssigkeit ist.

3. Verfahren nach Anspruch 2, wobei der Schritt des Isolierens der Lipoproteinpartikel in zwei oder mehrere Klassen und/oder Unterklassen umfasst:
Isolieren der HDL-Partikel aus nicht-HDL-Partikeln, wobei mindestens eines aus freiem Cholesterin und Phospholipid, die von isolierten HDL-Partikeln abgetrennt sind, gemessen wird, um die Menge der HDL-Partikel zu bestimmen.

4. Verfahren nach Anspruch 3, wobei die HDL-Partikel von den nicht-HDL-Partikeln durch Ausfällen mit einem Fällungsmittel, wie zum Beispiel Dextransulfat, isoliert werden.

5. Verfahren nach Anspruch 3, weiter umfassend:
Fraktionieren der isolierten HDL-Partikel in eine oder mehrere Unterklassen, wobei mindestens eines aus freiem Cholesterin und Phospholipid, die von jeder fraktionierten HDL-Partikelunterklasse abgetrennt sind, gemessen wird, um die Menge der fraktionierten HDL-Partikelunterklasse zu bestimmen, wobei gegebenenfalls die HDL-Unterklassen eine oder mehrere aus HDL-1, HDL-2 und HDL-3 umfassen.

6. Verfahren nach Anspruch 1 oder Anspruch 5, wobei einer oder beide der Isolierungsschritte oder des Fraktionierens mittels Ausfällen, Elektrophorese, Zentrifugation, Ultrazentrifugation, Benutzen eines grenzflächenaktiven Mittels, Benutzen eines Detergens oder einer Kombination davon ausgeführt wird, wobei das Fraktionieren gegebenenfalls durch Ausfällen in aufeinanderfolgenden Schritten ausgeführt wird, wobei jeweils eine HDL-Partikelunterklasse ausfällt.

7. Verfahren nach Anspruch 1, wobei das Isolieren der Lipoproteinpartikel durch Elektrophorese durchgeführt wird, um alle oder fast alle Klassen der Lipoproteinpartikel von den nicht-Lipoproteinbestandteilen in der biologischen Probe zu isolieren und um verschiedene Klassen oder Unterklassen der Lipoproteinpartikel gleichzeitig voneinander zu isolieren und

wobei gegebenenfalls das resultierende elektrophoretische Gel für jeden isolierten Lipoproteinpartikel zum Messen der Menge von mindestens einem aus freiem Cholesterin und Phospholipid in dem Lipoproteinpartikel benutzt wird, wodurch die Mengen aller isolierten Klassen oder Unterklassen der Lipoproteinpartikel gleichzeitig oder fast gleichzeitig bestimmt werden können und
wobei gegebenenfalls die gleichzeitig bestimmten Lipoproteinpartikel zwei oder mehrere verschiedene Klassen oder Unterklassen von Lipoproteinpartikeln umfassen oder wobei die gleichzeitig bestimmten Lipoproteinpartikel zwei oder mehrere aus HDL-P, VLDL-P, IDL-P und LDL-P umfassen.

8. Verfahren nach Anspruch 1, wobei der Bestimmungsschritt auf empirisch abgeleiteten Algorithmen basiert, die experimentell aus Bevölkerungsstudien bestimmt werden, welche die Mengen von mindestens einem aus freiem Cholesterin und Phospholipid mit der Lipoproteinpartikelanzahl in Beziehung setzen.

9. Verfahren nach Anspruch 1, wobei der Durchmesser oder die Radien der Lipoproteinpartikel vorbestimmt sind; und der Bestimmungsschritt auf der gemessenen Menge von mindestens einem aus freiem Cholesterin und Phospholipid und dem vorbestimmten Durchmesser oder Radien basiert, wobei gegebenenfalls der Durchmesser oder die Radien der Lipoproteinpartikel basierend auf theoretischen Schätzungen oder auf experimentellen Messungen der durchschnittlichen Größen der Klassen und Unterklassen der Lipoproteinpartikel in einem Individuum oder einer Bevölkerung an Individuen vorbestimmt werden.

**10.** Verfahren nach Anspruch 2(i), wobei der Isolierungsschritt der Lipoproteinpartikel in zwei oder mehrere Klassen und/oder Unterklassen umfasst:
Isolieren von LDL-Partikeln aus nicht-LDL-Partikeln, wobei mindestens eines aus freiem Cholesterin und Phospholipid, die von den isolierten LDL-Partikeln abgetrennt sind, gemessen wird, um die Menge der HDL-Partikel zu bestimmen; gegebenenfalls weiter umfassend:
Fraktionieren der isolierten LDL-Partikel in eine oder mehrere Unterklassen, wobei mindestens eines aus freiem Cholesterin und Phospholipid, die von jeder fraktionierten LDL-Partikelunterklasse abgetrennt sind, gemessen wird, um die Menge der fraktionierten LDL-Unterklassenpartikel zu bestimmen.

## Revendications

**1.** Procédé d'évaluation des quantités d'une ou plusieurs parmi des particules de lipoprotéine haute densité (HDL), de lipoprotéine basse densité (LDL), de lipoprotéine très basse densité (VLDL), de lipoprotéine de densité intermédiaire (IDL) et de lipoprotéine (a) (Lp(a)) dans un échantillon biologique, comprenant :

l'isolement des particules de lipoprotéine HDL, LDL, VLDL, IDL et Lp(a) des composants non lipoprotéiniques dans l'échantillon biologique, pour générer des particules de lipoprotéine HDL, LDL, VLDL, IDL et Lp(a) isolées ;
la séparation d'au moins l'un parmi le cholestérol et les phospholipides libres des particules de lipoprotéine HDL, LDL, VLDL, IDL et Lp(a) isolées ;
la mesure de la quantité de l'au moins un du cholestérol et des phospholipides libres qui a été séparée de l'une ou plusieurs des particules de lipoprotéine HDL, LDL, VLDL, IDL et Lp(a) isolées ; et
la détermination des quantités de la ou des plusieurs particules de lipoprotéine HDL, LDL, VLDL, IDL et Lp(a) sur base du diamètre ou du rayon des particules de lipoprotéine et la quantité mesurée de cholestérol ou de phospholipides libres.

**2.** Procédé selon la revendication 1,

(i) dans lequel le procédé comprend en outre, avant l'étape de séparation, l'isolement des particules de lipoprotéines en deux ou plusieurs classes et/ou sous-classes, dans lequel l'étape de séparation suivante est réalisée sur une ou plusieurs particules de lipoprotéine dans une ou plusieurs des classes et/ou sous-classes isolées,
(ii) dans lequel la séparation du cholestérol libre des particules de lipoprotéine HDL, LDL, VLDL, IDL et Lp(a) isolées est réalisée en soumettant les particules de lipoprotéine à une cholestérol oxydase, ou dans lequel une cholestérol estérase n'est pas utilisée pour séparer le cholestérol libre des particules de lipoprotéine HDL, LDL, VLDL, IDL et Lp(a) isolées, ce par quoi le cholestérol estérifié dans le cœur des particules de lipoprotéine n'est pas libéré sous forme de cholestérol libre,
(iii) dans lequel l'échantillon biologique est une matrice biologique humaine, une fraction de lipoprotéine humaine, un composant sanguin, de l'urine, du plasma, du sérum, du liquide synovial ou du liquide ascitique.

**3.** Procédé selon la revendication 2, dans lequel l'étape d'isolement des particules de lipoprotéine en deux ou plusieurs classes et/ou sous-classes, comprend :
l'isolement des particules HDL des particules non-HDL, dans lequel l'au moins un du cholestérol et des phospholipides libres séparés des particules HDL isolées est mesuré pour déterminer les quantités de particules HDL.

**4.** Procédé selon la revendication 3, dans lequel les particules HDL sont isolées des particules non-HDL par précipitation avec un agent de précipitation, tel que le sulfate de dextrane.

**5.** Procédé selon la revendication 3, comprenant en outre
le fractionnement des particules HDL isolées en une ou plusieurs sous-classes, dans lequel l'au moins un du cholestérol et des phospholipides libres séparés de chaque particule de sous-classe HDL fractionnée est mesuré pour déterminer les quantités de la particule de sous-classe HDL fractionnée,
le cas échéant, où les sous-classes HDL comprennent l'un ou plusieurs parmi HDL-1, HDL-2 et HDL-3.

**6.** Procédé selon la revendication 1 ou la revendication 5, dans lequel l'une ou les deux étapes d'isolement, ou de fractionnement, sont réalisées par précipitation, électrophorèse, centrifugation, ultracentrifugation, à l'aide d'un

tensioactif, à l'aide d'un détergent, ou une combinaison de ceux-ci,
le cas échéant dans lequel le fractionnement par précipitation est réalisé selon des étapes séquentielles, de précipitation d'une particule de sous-classe HDL à la fois.

**7.** Procédé selon la revendication 1, dans lequel l'isolement des particules de lipoprotéine est réalisé par électrophorèse pour isoler toutes ou presque toutes les classes de particules de lipoprotéine des composants non lipoprotéiniques dans l'échantillon biologique, et pour isoler différents classes ou sous-classes de particules de lipoprotéine les unes des autres simultanément, et,
le cas échéant, dans lequel le gel d'électrophorèse résultant pour chaque particule de lipoprotéine isolée est utilisé pour mesurer la quantité de l'au moins un parmi le cholestérol et les phospholipides libres dans la particules de lipoprotéine, où les quantités de toutes les classes ou sous-classes isolées des particules de lipoprotéine peuvent être déterminées simultanément ou presque simultanément, et,
le cas échéant, dans lequel les particules de lipoprotéine simultanément déterminées comprennent deux ou plusieurs classes ou sous-classes différentes de particules de lipoprotéine, ou dans lequel les particules de lipoprotéine déterminées simultanément comprennent deux ou plusieurs parmi HDL-P, VLDL-P, IDL-P, et LDL-P.

**8.** Procédé selon la revendication 1, dans lequel l'étape de détermination est basée sur des algorithmes dérivés empiriquement déterminés expérimentalement à partir d'études de population liant les quantités d'au moins l'un parmi le cholestérol et les phospholipides libres aux nombres de particules de lipoprotéine.

**9.** Procédé selon la revendication 1, dans lequel le diamètre ou le rayon des particules de lipoprotéine sont déterminés préalablement; et l'étape de détermination est basée sur la quantité mesurée d'au moins l'un parmi le cholestérol et les phospholipides libres et du diamètre ou rayon déterminé préalablement, le cas échéant dans lequel le diamètre ou rayon des particules de lipoprotéine sont déterminés préalablement sur base d'estimation théorique ou sont déterminés préalablement sur base de la mesure expérimentale des tailles moyennes des classes et sous-classes des particules de lipoprotéine chez un individu ou une population d'individus.

**10.** Procédé selon la revendication 2(i), dans lequel l'étape d'isolement des particules de lipoprotéine en deux ou plusieurs classes et/ou sous-classes, comprend :
l'isolement des particules LDL des particules non LDL, dans lequel l'au moins un parmi le cholestérol et les phospholipides libres séparés des particules LDL isolées est mesuré pour déterminer les quantités de particules LDL ;
le cas échéant comprenant en outre:
le fractionnement des particules LDL isolées en une ou plusieurs sous-classes, dans lequel l'au moins un du cholestérol et des phospholipides libres séparés de chaque particules de sous-classe LDL fractionnée est mesuré pour déterminer la quantité de la particule de sous-classe LDL fractionnée.

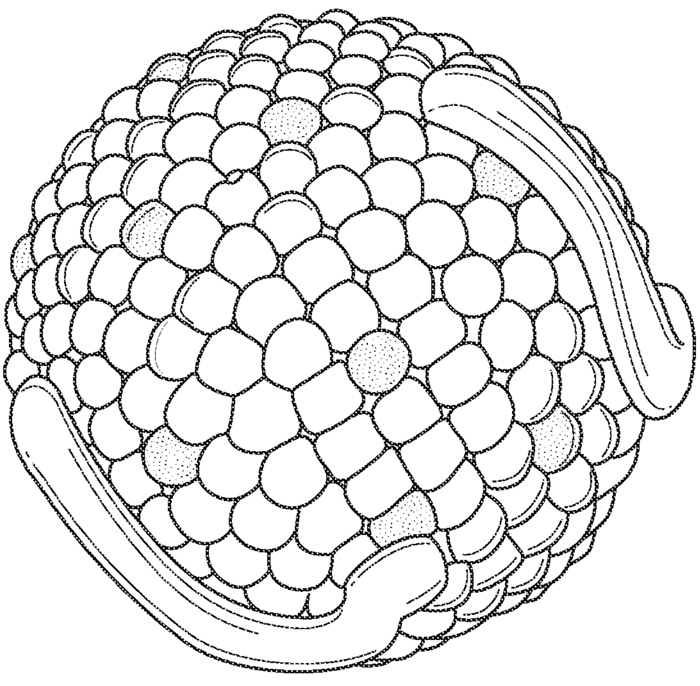

FIG. 1

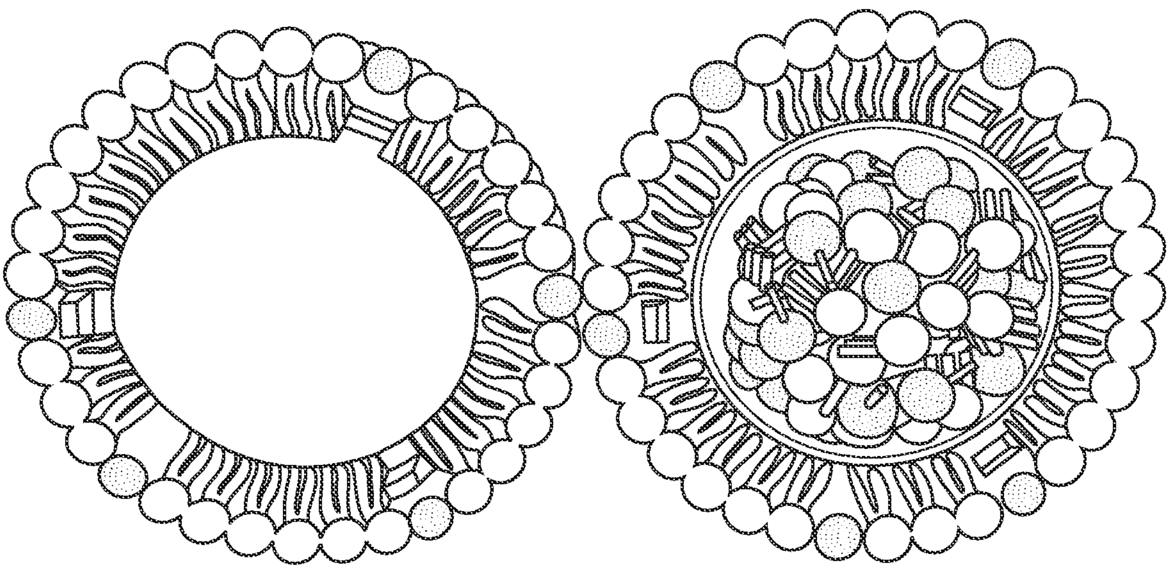

FIG. 2

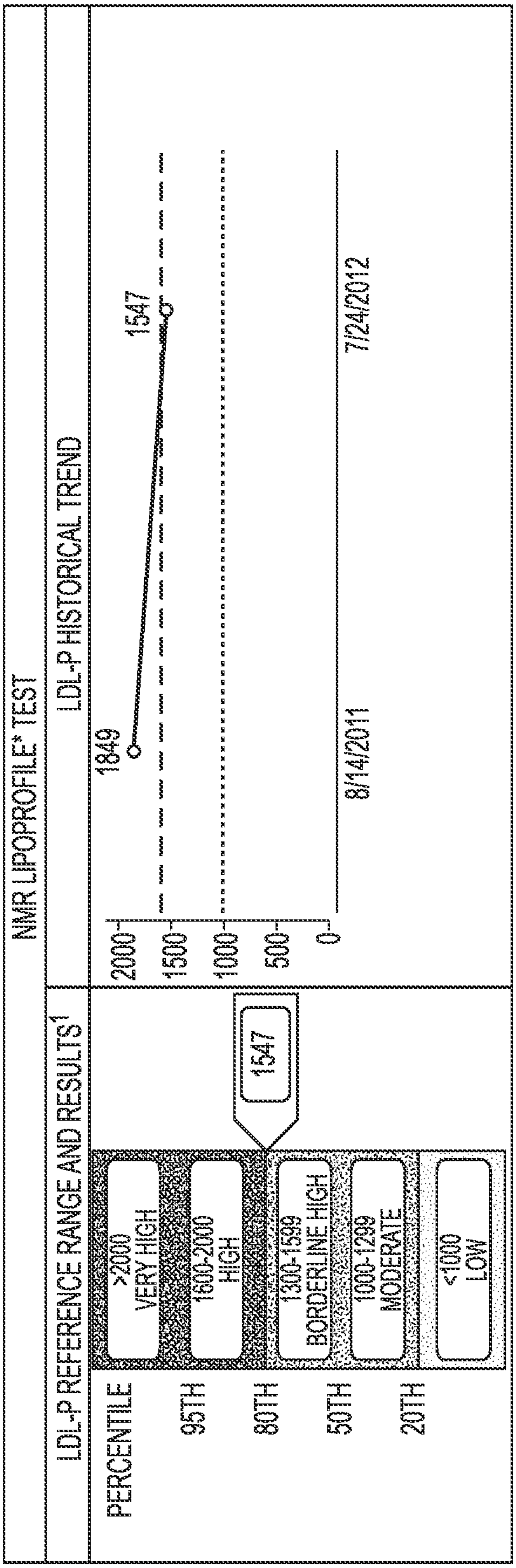
NMR LIPOPROFILE® TEST
LDL-P REFERENCE RANGE AND RESULTS[1]
PERCENTILE
95TH
80TH
50TH
20TH
>2000 VERY HIGH
1600-2000 HIGH
1300-1599 BORDERLINE HIGH
1000-1299 MODERATE
<1000 LOW
1547
LDL-P HISTORICAL TREND
2000
1500
1000
500
0
1849
1547
8/14/2011
7/24/2012

FIG. 3

| PARTICLE CONCENTRATION AND SIZE | | | | |
|---|---|---|---|---|
| | LABORATORY TEST | RESULT | PRECENTILE IN REFERENCE POPULATION[2]<br>HIGHER CVD RISK ⟷ LOWER CVD RISK | PREVIOUS RESULTS |
| HDL PARTICLES | HDL-P (TOTAL) µmol/L | 34.0 | LOW · 25TH (28.7) · 50TH (30.5) · 75TH (34.9) · HIGH<br>34 | 28.1 |
| SMALL LDL-P AND LDL SIZE ARE ASSOCIATED WITH CVD RISK, BUT NOT AFTER LDL-P IS TAKEN INTO ACCOUNT. | | | INSULIN RESISTANCE ⟷ INSULIN SENSITIVE | |
| LIPOPROTEIN MARKERS ASSOCIATED WITH INSULIN RESISTANCE AND DIABETES RISK | LARGE VLDL-P nmol/L | 1.7 | HIGH · 75TH (6.9) · 50TH (2.7) · 25TH (0.9) · LOW<br>1.7 | 1.1 |
| | SMALL VLDL-P nmol/L | 733 | HIGH · 75TH (839) · 50TH (527) · 25TH (117) · LOW<br>733 | 1026 |
| | LARGE HDL-P µmol/L | 3.2 | LOW · 25TH (3.1) · 50TH (4.8) · 75TH (7.3) · HIGH<br>3.2 | 2.1 |
| | VLDL SIZE nm | 40.9 | LARGE · 75TH (52.5) · 50TH (46.6) · 25TH (42.4) · SMALL<br>40.9 | 44.5 |
| | LDL SIZE nm | 21.1 | SMALL · 25TH (20.4) · 50TH (20.8) · 75TH (21.2) · LARGE<br>21.1 | 20.5 |
| | HDL SIZE nm | 8.5 | SMALL · 25TH (8.9) · 50TH (9.2) · 75TH (9.6) · LARGE<br>8.5 | 8.3 |
| | LP-IR SCORE* 0-100 | 49 | INSULIN RESISTANT · 75TH (63) · 50TH (45) · 25TH (27) · INSULIN SENSITIVE<br>49 | 57 |

**FIG. 3**
**CONTINUED**

CORRELATIONS

| | HDL3 | HDL-C | HDL PARTICLE NUMBER |
|---|---|---|---|
| HDL3 | 1.0000 | 0.8794 | 0.8175 |
| HDL-C | 0.8794 | 1.0000 | 0.6877 |
| HDL PARTICLE NUMBER | 0.8175 | 0.6877 | 1.0000 |

SCATTERPLOT MATRIX

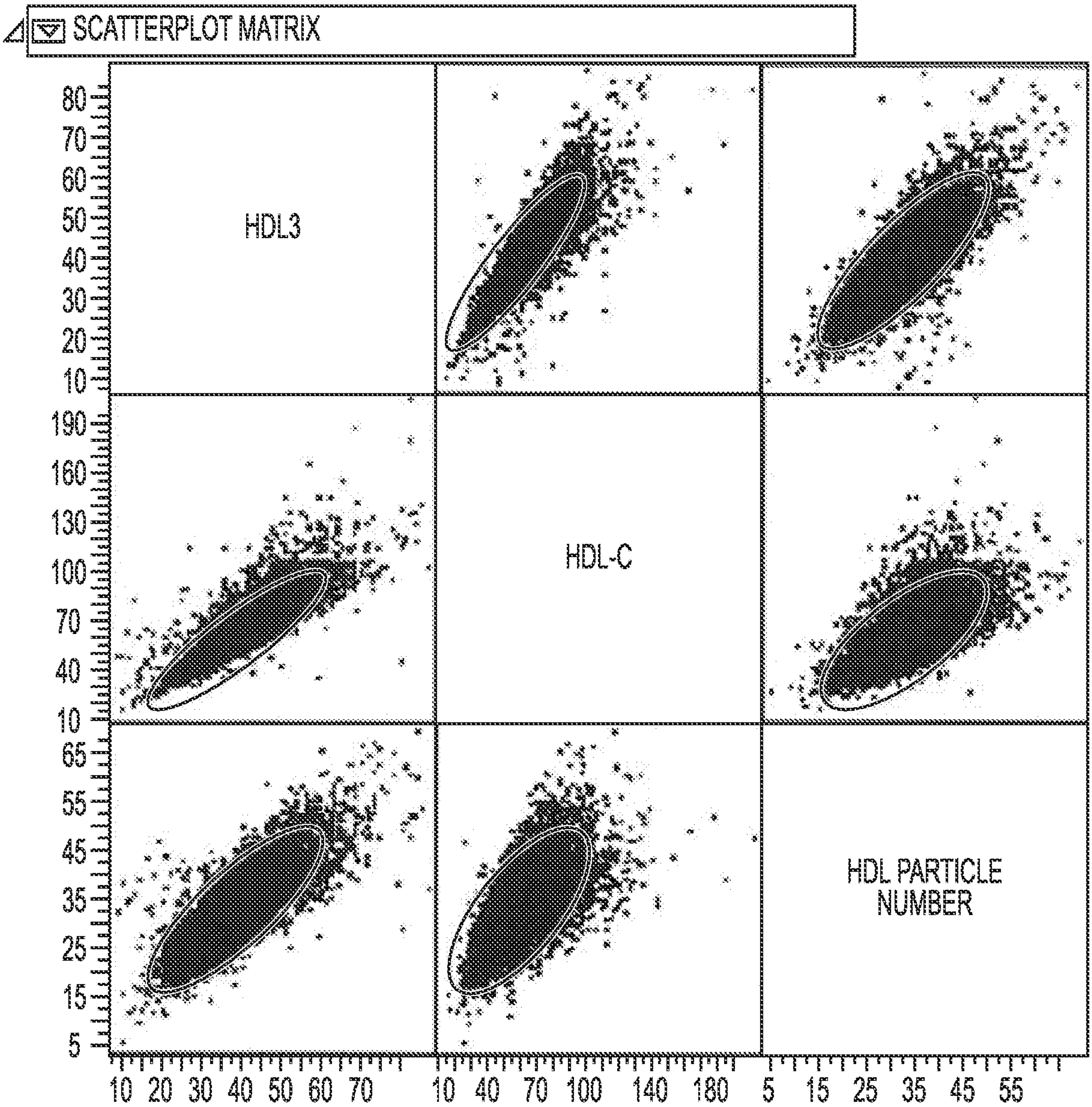


FIG. 4

BIVARIATE FIT OF HDL PARTICLE NUMBER BY HDL-C

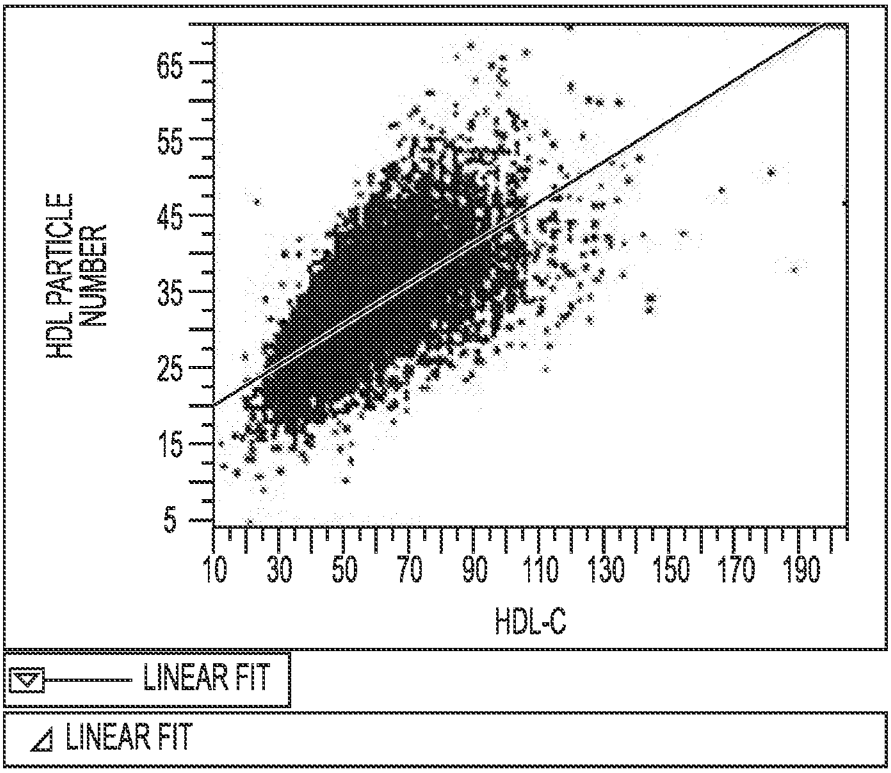


LINEAR FIT

LINEAR FIT

HDL PARTICLE NUMBER = 16.892106 + 0.274105*HDL-C

SUMMARY OF FIT

| | |
|---|---|
| RSQUARE | 0.472904 |
| RSQUARTE ADJ | 0.472857 |
| ROOT MEAN SQUARE ERROR | 4.969398 |
| MEAN OF RESPONSE | 32.56702 |
| OBSERVATIONS (OR SUM WGTS) | 11108 |

LACK OF FIT

ANALYSIS OF VARIANCE

| SOURCE | DF | SUM OF SQUARES | MEAN SQUARE | F RATIO |
|---|---|---|---|---|
| MODEL | 1 | 246064.39 | 246064 | 9964.173 |
| ERROR | 11106 | 274261.71 | 25 | PROB>F |
| C.TOTAL | 11107 | 520326.09 | | <.0001* |

PARAMETER ESTIMATES

| TERM | ESTIMATE | STD ERROR | t RATIO | PROB>\|t\| |
|---|---|---|---|---|
| INTERCEPT | 16.892106 | 0.163957 | 103.03 | <.0001* |
| HDL-C | 0.274105 | 0.002746 | 99.82 | <.0001* |

FIG. 5

BIVARIATE FIT OF HDL PARTICLE NUMBER BY HDL3

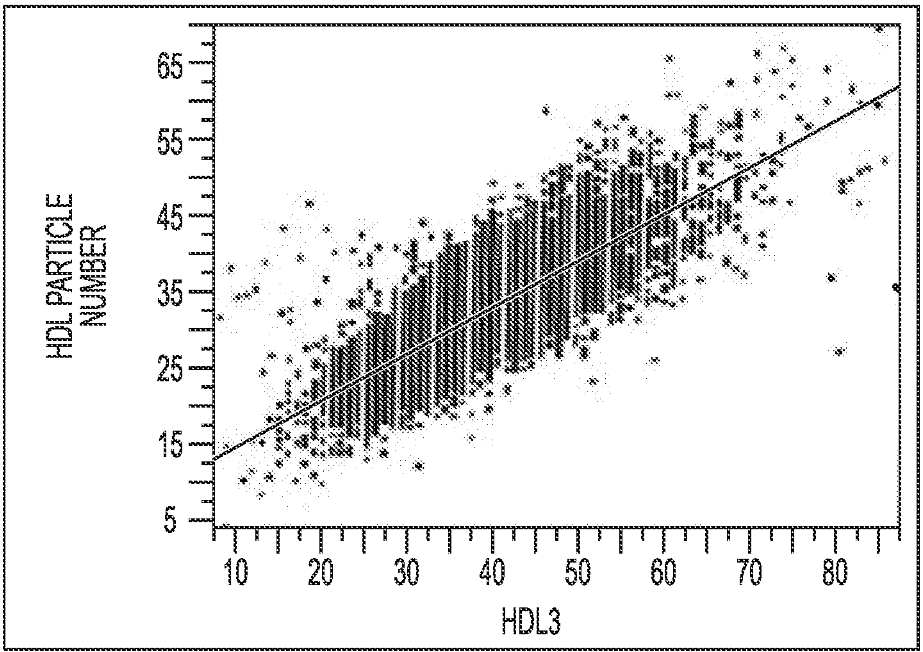


LINEAR FIT

LINEAR FIT

HDL PARTICLE NUMBER = 8.5542777 + 0.6202472*HDL3

SUMMARY OF FIT

| | |
|---|---|
| RSQUARE | 0.668283 |
| RSQUARTE ADJ | 0.668253 |
| ROOT MEAN SQUARE ERROR | 3.94224 |
| MEAN OF RESPONSE | 32.56702 |
| OBSERVATIONS (OR SUM WGTS) | 11108 |

LACK OF FIT

ANALYSIS OF VARIANCE

| SOURCE | DF | SUM OF SQUARES | MEAN SQUARE | F RATIO |
|---|---|---|---|---|
| MODEL | 1 | 347724.92 | 347725 | 22374.32 |
| ERROR | 11106 | 172601.17 | 16 | PROB>F |
| C.TOTAL | 11107 | 520326.09 | | <.0001* |

PARAMETER ESTIMATES

| TERM | ESTIMATE | STD ERROR | t RATIO | PROB>\|t\| |
|---|---|---|---|---|
| INTERCEPT | 8.5542777 | 0.164834 | 51.90 | <.0001* |
| HDL3 | 0.6202472 | 0.004147 | 149.58 | <.0001* |

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6479249 B
- US 20130078659
- US 20100227309
- US 61747505
- US 14426913
- US 20080038829 A1
- US 861829 A
- US 61651975
- US 61770406 B
- US 61779567
- US 61652608 B

### Non-patent literature cited in the description


- **SHEN et al.** Structure of Human Serum Lipoproteins Inferred from Compositional Analysis. *PNAS USA,* 1977, vol. 74 (3), 837-41
- **HAROLD E. BAYS et al.** Once-Daily Niacin Extended Release/Lovastatin Combination Tablet Has More Favorable Effects on Lipoprotein Particle Size and Subclass Distribution Than Atorvastatin and Simvastatin. *Preventive Cardiology,* 2003, vol. 6 (4), 179-88, 187
- **NADER RIFAI et al.** Handbook of Lipoprotein Testing. American Association for Clinical Chemistry, Inc, 2001
- **LORENZI et al.** Apolipoprotein A-I but not high-density lipoproteins are internalised by RAW macrophages: roles of ATP-binding cassette transporter AI and scavenger receptor. *BIJMol Med.,* 2008, vol. 86, 171-183
- **MEYERS et al.** Pharmacologic elevation of high-density lipoproteins: recent insights on mechanism of action and atherosclerosis protection. *Curr Opin Cardiol.,* 2004, vol. 19 (4), 366-373
- **DE BACKER et al.** European Guidelines on Cardiovascular Disease Prevention in Clinical Practice. Third Joint Task Force of European and other Societies on Cardiovascular Disease Prevention in Clinical Practice. *Eur Heart J,* 2003, vol. 24, 1601-1610
- **WALLDIUS ; JUNGNER.** Apolipoprotein B and Apolipoprotein A-I: Risk Indicators of Coronary Heart Disease and Targets for Lipid-modifying Therapy. *J Intern Med,* 2004, vol. 255 (2), 188-205
- **WALLDIUS et al.** The apoB/apoA-I ratio: A Strong, New Risk Factor for Cardiovascular Disease and a Target for Lipid-Lowering Therapy- A Review of the Evidence. *J Intern Med.,* 2006, vol. 259 (5), 493-519
- **YUSUF et al.** Effect of Potentially Modifiable Risk Factors Associated with Myocardial Infarction in 52 Countries (the TNTERHEART Study): Case-control Study. *Lancet,* 2004, vol. 364, 937-52
- **KANNEL et al.** Cholesterol in the Prediction of Atherosclerotic Disease. *Ann Intern Med,* 1979, vol. 90, 85-91
- **JEYARAJAH et al.** Lipoprotein Particle Analysis by Nuclear Magnetic Resonance Spectroscopy. *Clin Lab Med,* 2006, vol. 26, 847-70
- **WALLDIUS ; JUNGNER.** Apolipoprotein B and Apolipoprotein A-I: Risk Indicators of Coronary Heart Disease and Targets for Lipid- modifying Therapy. *J Intern Med,* 2004, vol. 255 (2), 188-205
- **WALLDIUS et al.** The apoB/apoA-I ratio: A Strong, New Risk Factor for Cardiovascular Disease and a Target for Lipid-Lowering Therapy-A Review of the Evidence. *J Intern Med.,* 2006, vol. 259 (5), 493-519
- **WALLDIUS et al.** Stroke Mortality and the Apo B/Apo A-I Ratio: Results of the AMORIS Prospective Study. *J Intern Med.,* 2006, vol. 259, 259-66
- **YUSUF et al.** Effect of Potentially Modifiable Risk Factors Associated with Myocardial Infarction in 52 Countries (the INTERHEART Study): Case-control Study. *Lancet,* 2004, vol. 364, 937-52
- **YUSUF et al.** Obesity and the risk of myocardial infarction in 27,000 participants from 52 countries: a case-control study. *Lancet,* 2005, vol. 366, 1640-9
- **FRIED et al.** The Cardiovascular Health Study: Design and Rationale. *Ann. Epidemiol.,* 1991, vol. 3, 263-76
- REMINGTON, THE SCIENCE AND PRACTICE OF PHARMACY. Mack Publishing Company, 2005

- **HASKELL et al.** Effects of Intensive Multiple Risk Factor Reduction on Coronary Atherosclerosis and Clinical Cardiac Events in Men and Women with Coronary Artery Disease. *Circulation,* 1994, vol. 89 (3), 975-990
- **ORNISH et al.** Intensive Lifestyle Changes for Reversal of Coronary Heart Disease. *JAMA,* 1998, vol. 220 (23), 2001-2007
- **WISTER et al.** One-year Follow-up of a Therapeutic Lifestyle Intervention Targeting Cardiovascular Disease Risk. *CMAJ,* 2007, vol. 177 (8), 859-865
- LDL Particle concentration and size as determined by NMR spectroscopy as predictors of CVD in Women. *Circulation,* 2002, vol. 106, 1930-1937
- Direct determination of lipoprotein particle sizes and concentrations by Ion Mobility Analysis. *Clin Chem,* 2008, vol. 54 (8), 1307-1316
- Tech Briefs. Rapid, Simple Laser-Light Scattering method for HDL particle sizing in whole plasma. *ClinChem,* 2004, 032383
- Advanced Lipoprotein Testing and subfractionation are not (yet) ready for routine clinical use. (provides references for gradient gel and ultracentrifugation protocols). *Circulation,* 2009, vol. 119, 2396-2404